(19) Europäisches Patentamt
European Patent Office
Office européen des brevets

(11) **EP 4 512 334 A1**

(12) **EUROPEAN PATENT APPLICATION**
published in accordance with Art. 153(4) EPC

(43) Date of publication:
26.02.2025 Bulletin 2025/09

(51) International Patent Classification (IPC):
$A61B\ 5/18^{(2006.01)}$     $A61B\ 5/024^{(2006.01)}$
$A61M\ 21/00^{(2006.01)}$     $G06F\ 17/10^{(2006.01)}$
$E02D\ 29/045^{(2006.01)}$     $B60W\ 40/08^{(2012.01)}$

(21) Application number: 23791348.8

(22) Date of filing: 21.04.2023

(86) International application number:
PCT/CN2023/089754

(87) International publication number:
WO 2023/202696 (26.10.2023 Gazette 2023/43)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 22.04.2022 CN 202210430058

(71) Applicant: Beijing University of Technology
Beijing 100124 (CN)

(72) Inventors:
• HU, Jiangbi
  Beijing 100124 (CN)
• GAO, Xiaojuan
  Beijing 100124 (CN)
• GUO, Yunpeng
  Beijing 100124 (CN)

(74) Representative: FDST Patentanwälte
Nordostpark 16
90411 Nürnberg (DE)

(54) **METHOD AND APPARATUS FOR SETTING FATIGUE AROUSAL SECTION IN TUNNEL, AND ROAD TUNNEL**

(57) In the disclosure provided are a method and device for arranging a fatigue arousal section in a tunnel, as well as a road tunnel. The method for arranging the fatigue arousal section in the tunnel comprises: determining a plurality of colors and luminance of fatigue arousal section; based on a plurality of samples, generating a plurality of arousal duration models in which the drivers' arousal duration changes with stimulus quantity at multiple different vehicle speeds, wherein the samples comprise arousal durations of different drivers under different stimulus quantities at the plurality of colors and luminance; determining length and spacing of fatigue arousal section to be arranged according to a specified speed of the tunnel, using the arousal duration models; and arranging one or more fatigue arousal sections in the tunnel based on the determined length and spacing of the fatigue arousal section. In the disclosure, there is support provided for arranging effective fatigue arousal sections in various long expressway tunnels, thereby meeting the physiological and psychological needs of drivers for safe and comfortable driving in various long tunnels.

FIG. 1

EP 4 512 334 A1

## Description

The application claims the priority of Chinese patent application 202210430058.4 filed on April 22, 2022, the entire contents of which are incorporated herein by reference.

## TECHNICAL FIELD

The application relates to the technical field of roadway lighting technology, and in particular to, method and device for arranging fatigue arousal section in tunnel. Furthermore, the application also relates to a relevant road tunnel.

## BACKGROUND

The expressway long tunnel possesses distinct characteristics such as confined spaces, high degree of enclosure, monotonous tunnel sidewalls, low luminance in the mediate section, high noise levels, and slow dispersion of smoke and dust. The spatial constraints within the tunnel structure also impact the driving environment. During extended journeys through long tunnels, drivers are exposed to repetitive and monotonous information for a long period, which may lead to a decline in physiological functions, resulting in drivers experiencing delayed reactions, diminished perception, reduced consciousness, decreased alertness, and psychological distress, gradually leading to driving fatigue. Fatigued drivers are more prone to engage in risky driving behaviors, such as speeding and reckless maneuvers, upon exiting the tunnel, posing safety risks to traffic.

Currently, there are proposals to alleviate driving fatigue within these long tunnels by arranging fatigue arousal sections. However, these existing solutions often rely on empirical data for arranging such sections. Even when some schemes incorporate simple simulation scenarios, they struggle to accurately simulate the actual impact of tunnel lighting conditions, such as luminance and color temperature, on driving safety. As a result, the arranged fatigue arousal sections often fail to effectively rouse drivers from their fatigue-induced state.

To address the issue and ensure that the fatigue arousal sections in highway tunnels, particularly in the expressway long tunnels cater to the physiological and psychological needs of drivers for safe and comfortable travel, it is needed to explore methods for calculating parameters for setting up fatigue arousal sections or devising solutions for their implementation, so as to facilitate the widespread arrangement of effective fatigue arousal sections in various expressway long tunnels, thereby meeting the physiological and psychological requirements of drivers during travel through these long tunnels.

The content described in the background is only for the purpose of understanding the relevant technology in the field, and should not be interpreted as an admission to the prior art.

## SUMMARY

The embodiments of the disclosure intend to provide a method and device for arranging a fatigue arousal section in a tunnel, as well as related road tunnels, which may provide support for arranging effective fatigue arousal sections in various expressway long tunnels, thereby meeting the physiological and psychological needs of drivers for safe and comfortable driving in various long tunnels.

In an embodiment of the disclosure, a method for arranging a fatigue arousal section in a tunnel is provided, which comprises:

> S130: determining a plurality of colors and luminance of fatigue arousal section;
> S160: based on a plurality of fifth samples, generating a plurality of arousal duration models in which the drivers' arousal duration changes with stimulus quantity at multiple different vehicle speeds, wherein the fifth samples comprise arousal durations of different drivers under different stimulus quantities at the plurality of colors and luminance;
> S170: determining length and spacing of fatigue arousal section to be arranged according to a specified speed of the tunnel, using the arousal duration models; and
> S180: arranging one or more fatigue arousal sections in the tunnel based on the determined length and spacing of the fatigue arousal section.

In an embodiment of the disclosure, each of the arousal duration models has a first plateau segment where the arousal duration remains roughly constant with changing stimulus quantity, a second rising segment where the arousal duration increases with increasing stimulus quantity, and a third plateau segment where the arousal duration remains roughly constant with changing stimulus quantity.

In an embodiment of the disclosure, each of the said arousal duration models is shown as follows:

$$T_i = \begin{cases} t0 & q0 \le Q_i < q1 \\ t1 - \dfrac{a}{1 + (\dfrac{Q_1}{b})^c} & q1 \le Q_i < q2 \\ t2 & Q_i \ge q2 \end{cases}$$

where T is the arousal duration, Q is the stimulus quantity, t0, t1, t2, a, b, c are the model parameters, q0, q1, q2 are the endpoint values of the stimulus quantity range, i refers to the serial number of the arousal duration model, the model parameters and endpoint values are different according to the arousal duration model.

Preferably, the plurality of arousal duration models are respectively represented as follows:

① for a first speed

$$T_1 = \begin{cases} 54.97502 & 8.84 \le Q_1 < 10.43 \\ 147.976 - \dfrac{95.24837}{1 + (\dfrac{Q_1}{12.2655})^{8.67823}} & 10.43 \le Q_1 < 16.99 \\ 146.6 & Q_1 \ge 16.99 \end{cases}$$

where $T_1$- driver's arousal duration after the fatigue arousal section at the first speed;
$Q_1$- the stimulus quantity of fatigue arousal section at the first speed;

② for a second speed

$$T_2 = \begin{cases} 64.13252 & 9.42 \le Q_2 < 13.877 \\ 172.6251 - \dfrac{111.1144}{1 + (\dfrac{Q_2}{16.31935})^{8.67823}} & 13.877 \le Q_2 < 22.608 \\ 171.02 & Q_2 \ge 22.608 \end{cases}$$

where $T_2$- driver's arousal duration after the fatigue arousal section at the second speed;
$Q_2$- the stimulus quantity of fatigue arousal section at the second speed;

③ for a third speed

$$T_3 = \begin{cases} 76.95752 & 17.64 \le Q_3 < 25.987 \\ 207.1461 - \dfrac{133.3347}{1 + (\dfrac{Q_2}{30.5598})^{8.67823}} & 25.987 \le Q_3 < 42.336 \\ 205.22 & Q_3 \ge 42.336 \end{cases}$$

where T3 -- driver's arousal duration after the fatigue arousal section at the third speed;
Q3 - the stimulus quantity of fatigue arousal section at the third speed.

In an embodiment of the disclosure, the step S170 comprises:

S171: determining the arousal duration model corresponding to the specified speed;

S172: obtaining the arousal duration and minimum stimulus quantity corresponding to the third plateau segment of the determined arousal duration model;

S173: determining a first length of the fatigue arousal section based on the minimum stimulus quantity corresponding to the third plateau segment, the color, and the luminance; and

S174: determining a first spacing of the fatigue arousal section based on the arousal duration.

In an embodiment of the disclosure, the step S170 further comprises:

S175: obtaining the length of the tunnel; and

S176: based on the tunnel length, the first length, and the first spacing, determining a second length and a second spacing for the end-portion fatigue arousal section using the determined arousal duration model.

In the embodiment of the disclosure, the step S180 comprises: arranging middle-portion fatigue arousal sections in the tunnel's middle portion based on the first length and the first spacing, and arranging an end-portion fatigue arousal section in the tunnel's end section based on the second length and second spacing.

In an embodiment of the disclosure, the step S160 comprises:

S161: based on the plurality of fifth samples obtained at a reference vehicle speed, generating a reference arousal duration model depicting how the driver's arousal duration changes with stimulus quantity at the reference vehicle speed, preferably the reference speed is zero, i.e., in a stationary state; and

S162: processing the reference arousal duration model using regression analysis algorithms to generate the plurality of arousal duration models corresponding to the multiple different vehicle speeds.

In an embodiment of the disclosure, the method further comprises:

S150: based on a plurality of fourth samples, generating an arousal level model in which the driver's arousal level changes with stimulus quantity, wherein the fourth samples comprise the arousal levels of different drivers at different stimulus quantities with the plurality of colors and luminance.

In the embodiment of the disclosure, the step S160 comprises: constraining the plurality of arousal duration models using a minimum stimulus quantity determined by the arousal level model.

In an embodiment of the disclosure, the method further comprises:

S140: obtaining third experimental data for generating the fourth and fifth samples, comprising:

S141: instructing one of multiple experimental drivers to visually recognize the lighting environment in a middle portion of the tunnel for a fourth predetermined duration to collect fourth heart rate variability data in a non-stimulated state;

S142: stimulating the experimental driver to induce arousal by simulating a fatigue arousal section with one of the plurality of colors and the luminance for one of multiple fifth predetermined durations, and collecting fifth heart rate variability data in the stimulated state;

S143: deactivating the simulated fatigue arousal section to allow the experimental driver to resume visual recognition in the lighting environment in the middle portion of the tunnel for a sixth predetermined duration, and collecting sixth heart rate variability data in the aroused state;

S144: Repeating steps S142 and S143 for each fifth predetermined duration;

S145: Repeating steps S141 to S144 for each experimental driver; and

S146: Repeating steps S141 to S145 for each color.

In the embodiment of the disclosure, the fourth samples are obtained from the fourth and fifth heart rate variability data. In the embodiment of the disclosure, the fifth samples are obtained at least from the sixth heart rate variability data.

In an embodiment of the disclosure, the step S130 comprises:

S131: obtaining second experimental data for generating third samples;

S132: processing the second experimental data using an arousal level quantification model to obtain the third samples, wherein the third samples comprise the arousal levels of different drivers under different colors;

S133: determining an arousal level threshold based on the third samples; and

S134: selecting a plurality of colors based on the determined luminance and the arousal level threshold.

In an embodiment of the disclosure, the step S131 comprises:

S1311: instructing one of multiple experimental drivers to visually recognize the lighting environment in the middle portion of the tunnel for a first predetermined duration to collect first heart rate variability data in a non-stimulated state;

S1312: stimulating the experimental driver to induce arousal by simulating a fatigue arousal section using one of the colors from a color library and one of the luminance from a luminance group for a second predetermined duration, and collecting second heart rate variability data in the stimulated state;

S1313: deactivating the simulated fatigue arousal section to allow the experimental driver to resume visual recognition in the lighting environment in the middle portion of the tunnel for a third predetermined duration;

S1314: repeating steps S1311 to S1313 for each color in the color library;

S1315: repeating steps S1311 to S1314 for each experimental driver; and

S1316: repeating steps S1311 to S1315 for each luminance in the luminance group.

In the embodiment of the disclosure, the third samples are obtained based on the first and second heart rate variability data, and the number of colors in the color library is greater than or equal to the number of the selected plurality of colors.

In an embodiment of the disclosure, the method further comprises:

S110: determining a starting arrangement position of the fatigue arousal section according to a determined driving fatigue threshold and the specified speed.

In the embodiment of the disclosure, the step S180 comprises: arranging one or more fatigue arousal sections in the tunnel based on the starting arrangement position, the length and the spacing of the fatigue arousal section.

In an embodiment of the disclosure, the step S110 comprises:

S111: determining length of at least one entrance portion;

S112: determining length of at least one transition portion;

S113: determining a driving distance when the driver experiences fatigue in the middle portion of the tunnel based on the driving fatigue threshold and the specified speed; and

S114: determining the starting arrangement position based on the length of the at least one entrance portion, the length of the at least one transition portion, and the driving distance.

Preferably, the model of driving fatigue level versus heart rate variability is represented as follows:

$$A_{ij} = \left[ \left( \frac{LF}{HF} \right)_{ij} - \left( \frac{LF}{HF} \right)_i \right] / \left( \frac{LF}{HF} \right)_i$$

where $A_{ij}$ - driving fatigue of $i^{th}$ driver at driving time j in the tunnel;

$\left( \dfrac{LF}{HF} \right)_{ij}$ - the HRV value of $i^{th}$ driver at the $j^{th}$ moment while driving in the tunnel;

$\left( \dfrac{LF}{HF} \right)_i$ - the HRV value of $i^{th}$ driver in calm state.

Preferably, the multiple fatigue level models are represented as follows:

① for a first speed

$$A = 1.80869 + \frac{163.00287}{104.6879 \sqrt{\dfrac{\pi}{2}}} * e^{-2\left( \frac{t - 146.7103}{104.6879} \right)^2}$$

② for a second speed

$$A = 1.91254 + \frac{181.81534}{124.46489 \sqrt{\dfrac{\pi}{2}}} * e^{-2\left( \frac{t - 174.20238}{124.46489} \right)^2}$$

③ for a third speed

$$A = 2.01221 + \frac{192.91589}{146.19899\sqrt{\dfrac{\pi}{2}}} * e^{-2\left(\frac{t-226.83989}{146.19899}\right)^2}$$

where A - the driving fatigue level of drivers in the tunnel environment;
t - the driving time of the drivers in the tunnel environment.

In an embodiment of the disclosure, the step S113 comprises:

S1132: determining the driving fatigue threshold based on a plurality of first samples according to a given model of driving fatigue level versus heart rate variability, wherein the first samples comprise heart rate variability of different drivers driving in a calm state and when feeling fatigued in the tunnel environment;
S1133: generating a plurality of fatigue level models depicting changes in drivers' driving fatigue level over time at different vehicle speeds, based on a plurality of second samples, wherein the second samples comprise driving fatigue levels of different drivers at different times when driving in the tunnel environment at different vehicle speeds; and
S1134: determining the driving distance when the driver experiences fatigue in the middle portion of the tunnel using the fatigue level model, based on the driving fatigue threshold and the specified speed.

In an embodiment of the disclosure, the step S113 further comprises:
S1131: obtaining first experimental data for generating the first and second samples, specifically comprising:

S11311: collecting heart rate variability data of one of multiple experimental drivers in a calm state;
S11312: instructing the driver to drive the vehicle into the tunnel at one of a plurality of experimental speeds, and collecting the driver's heart rate variability data in real time;
S11313: recording the moment when the driver feels fatigued during the driving process, for determining the heart rate variability data of the driver at that moment; and
S11314: repeating steps S11311 to S11313 for each experimental driver and each experimental speed.

In an embodiment of the disclosure, the method further comprises:
S120: determining an arrangement height of the fatigue arousal section based on the determined driver's eye height and the specified speed;
The step S180 comprises: arranging the one or more fatigue arousal sections in the tunnel based on the arrangement height, the length, and the spacing of the fatigue arousal section.
In an embodiment of the disclosure, the fatigue arousal section comprises wall washer lights alternating in multiple colors installed on both sides of the road, with the wall washer lights having the specified luminance.
In the embodiment of the disclosure, a device for arranging a fatigue arousal section in a tunnel is provided, comprising:

a first determination unit, configured to determine a plurality of colors and luminance for the fatigue arousal section;
a generation unit, configured to generate a plurality of arousal duration models based on a plurality of fifth samples in which the drivers' arousal duration changes with stimulus quantity at a plurality of different vehicle speeds, the fifth samples comprising arousal durations of different drivers under different stimulus quantities at the plurality of colors and luminance;
a second determination unit, configured to determine length and spacing of the fatigue arousal section to be arranged according to the specified speed of the tunnel using the arousal duration models; and
an arrangement unit, configured to arrange one or more fatigue arousal sections in the tunnel based on the determined length and spacing of the fatigue arousal section.

In the embodiment of the disclosure, a road tunnel is provided, which comprises fatigue arousal sections arranged according to the method for arranging a fatigue arousal section in a tunnel in any one of the embodiments of the disclosure.
By means of the arrangement method, calculation method and devices provided in the embodiments of the disclosure, the arrangements and parameters of fatigue arousal sections that meet drivers' physiological and psychological needs from the perspective of their physiological and psychological needs for safe and comfortable driving in expressway long tunnels are obtained, thereby realizing a safe, comfortable, reasonable, and effective technique for arranging fatigue arousal sections in expressway long tunnels to arouse drivers.

The optional features and other effects of the embodiments of the disclosure are partly described below, and partly will be understandable after reading the disclosure.

## BRIEF DESCRIPTION OF DRAWINGS

The embodiments of the disclosure will be described below in details with reference to the accompanying drawings, in which the elements as shown are not limited by the scale shown in the drawings and the same or similar reference numerals in the drawings denote the same or similar elements, wherein:

FIG. 1 shows a first exemplary flowchart of the arranging method according to an embodiment of the disclosure;
FIG. 2 shows a second exemplary flowchart of the arranging method according to an embodiment of the disclosure;
FIG. 3 shows a third exemplary flowchart of the arranging method according to an embodiment of the disclosure;
FIG. 4 shows a fourth exemplary flowchart of the arranging method according to an embodiment of the disclosure;
FIG. 5 shows a fifth exemplary flowchart of the arranging method according to an embodiment of the disclosure;
FIG. 6 shows a sixth exemplary flowchart of the arranging method according to an embodiment of the disclosure;
FIG. 7 shows a seventh exemplary flowchart of the setting method according to an embodiment of the disclosure;
FIG. 8 shows an eighth exemplary flowchart of the arranging method according to an embodiment of the disclosure;
FIG. 9 shows a ninth exemplary flowchart of the arranging method in accordance with an embodiment of the disclosure;
FIG. 10 shows a tenth exemplary flowchart of the arranging method according to an embodiment of the disclosure;
FIG. 11 shows a diagram illustrating the variation pattern of the average driving fatigue level over time according to embodiments of the disclosure;
FIG. 12 shows a schematic diagram of a human eye's adaptive luminance curve according to embodiments of the disclosure;
FIG. 13A to 13C show diagrams illustrating the areas and heights of potential visual stimuli at different operating speeds according to embodiments of the disclosure;
FIG. 13D shows a schematically perspective view for calculating the area and height of potential visual stimuli according to embodiments of the disclosure;
FIG. 14A shows a schematic figure illustrating an the experimental field for an embodiment of the disclosure;
FIG. 14B shows a schematic figure illustrating an experimental field for an embodiment of the disclosure;
FIG. 15 shows a schematic diagram illustrating the subjective evaluation ranking of stimulus intensity for various colors according to an embodiment of the disclosure;
FIG. 16 shows a schematic diagram illustrating the arousal levels after stimuli from the fatigue arousal sections with different colors according to an embodiment of the disclosure;
FIG. 17 shows a schematic diagram illustrating the arousal levels after stimuli from the fatigue arousal sections with different sidewall luminance according to an embodiment of the disclosure;
FIG. 18 shows a schematic diagram of the relationship between the fatigue arousal section and each factor indicator according to the embodiment of the disclosure;
FIG. 19 shows a schematic diagram of the fitting curve of the stimulus quantity and the arousal level according to the embodiment of the disclosure;
FIG. 20 shows the variation pattern of heart rate variability data after arousing by the different stimulus levels according to the embodiment of the disclosure;
FIG. 21 shows a schematic diagram showing the relationship between the arousal duration and the stimulus quantity of the fatigue arousal section according to the embodiment of the disclosure; and
FIG. 22 shows a schematic arrangement of a fatigue arousal section in a long tunnel according to an embodiment of the disclosure.

## DETAILED DESCRIPTION OF SPECIFIC EMBODIMENTS

In order to make the objectives, technical solutions and advantages of the disclosure more apparent, the disclosure will be further described in detail below with reference to the specific embodiments and accompanying drawings. The exemplary embodiments of the disclosure and their descriptions are as explanations instead of limitation to the disclosure.

The term "comprise" used herein and its variations indicate an open-ended inclusion, i.e. "comprising but not limited to". Unless otherwise specified, the term "or" means "and/or". The term "based on" means "at least partially based on". The terms "an exemplary embodiment" and "one embodiment" represent "at least one example embodiment". The term "another embodiment" means "at least one other exemplary embodiment". To facilitate the understanding of this specification, the sequential terms "first", "second" and the like are used herein to distinguish different elements/items-s/objects and do not imply a specific order or importance of the different elements/items/objects. In particular, the method steps expressed by the terms "first", "second" and the like are not used to represent the order of method steps. Elements/items/objects denoted by terms such as "first", "second" and the like in an earlier order are not necessarily essential technical features of an embodiment where that embodiment includes elements/items/objects represented by a later sequential order. For example, the description of a "fifth sample" in an embodiment does not imply that the embodiment must comprise the first through fourth samples.

As mentioned above, the current methods often rely on empirical data or simple simulation scenarios to arrange fatigue arousal sections, which fail to effectively arrange fatigue arousal sections for effectively arousing drivers in actual scenarios.

Accordingly, the embodiments of the disclosure intend to provide a method and device for arranging a fatigue arousal section in a tunnel, a method and device for calculating arrangement parameters of fatigue arousal sections in tunnels, and related road tunnels, which may provide support for arranging effective fatigue arousal sections in various expressway long tunnels, thereby meeting the physiological and psychological needs of drivers for safe and comfortable driving in various long tunnels.

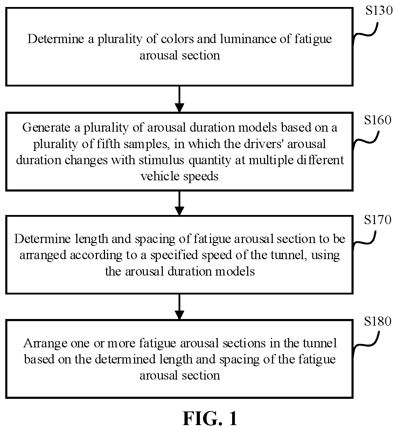

The specific embodiments of the disclosure will be described in detail below with reference to the accompanying drawings. As shown in FIG. 1, a method for arranging fatigue arousal sections in tunnels is illustrated, which may comprise:

S130: determining a plurality of colors and luminance of fatigue arousal section;
S160: based on a plurality of fifth samples, generating a plurality of arousal duration models in which the drivers' arousal duration changes with stimulus quantity at multiple different vehicle speeds;
S170: determining length and spacing of fatigue arousal section to be arranged according to a specified speed of the tunnel, using the arousal duration models; and
S180: arranging one or more fatigue arousal sections in the tunnel based on the determined length and spacing of the fatigue arousal section.

In the embodiment of the disclosure, the fifth samples comprise arousal duration of different drivers under different stimulus quantities at the plurality of colors and luminance.

In this embodiment, by providing the plurality of quantitative models that depict the relationship between stimulus quantity and arousal duration, it is possible to achieve a scientifically effective solution to arranging fatigue arousal sections based on the specified speed of the tunnel. This approach ensures that drivers' physiological and psychological needs for safe and comfortable driving can be met in various tunnels while effectively conserving energy.

In the embodiment of the disclosure, the prescribed vehicle speed may be broadly interpreted. In one embodiment, the specified speed refers to the speed limit of the tunnel or, when there are multiple speed limits, one of the speed limits, such as the speed limit for the majority of the tunnel. In another embodiment, the specified speed may be a speed set by those skilled in the art when implementing the embodiment of the disclosure according to the teachings of the disclosure, for example, a speed set based on the speed limit further in consideration with a safety margin; or, an average of the multiple speed limits. In the embodiment of the disclosure, the " according to a specified speed of the tunnel" may be one same specified speed for the whole tunnel, such as the previously mentioned speed limit or one of the speed limits, the speed with a safety margin, or the average speed. Alternatively, it may refer to different speeds set for different areas of the tunnel. For example, according to different speed limits for different sections of the tunnel, corresponding arousal duration models may be applied.

In further embodiments, each of the arousal duration models has a first plateau segment where the arousal duration remains roughly constant with changing stimulus quantity, a second rising segment where the arousal duration increases with increasing stimulus quantity, and a third plateau segment where the arousal duration remains roughly constant again with changing stimulus quantity.

In a specific embodiment, each of the said arousal duration models may be represented by the following equation:

$$T_i = \begin{cases} t0 & q0 \le Q_i < q1 \\ t1 - \dfrac{a}{1 + (\dfrac{Q_1}{b})^c} & q1 \le Q_i < q2 \\ t2 & Q_i \ge q2 \end{cases}$$

where T is the arousal duration, Q is the stimulus quantity, *t0, t1, t2, a, b, c* are the model parameters, *q0, q1, q2* are the endpoint values of the stimulus quantity range, i refers to the serial number of the arousal duration model, the model parameters and endpoint values are different according to the different arousal duration models.

In a preferred embodiment, a plurality of arousal duration models may be shown as follows:

① Speed 1 (a specific speed 1 (e.g. 60km/h) or a speed range 1 (e.g. 60 km/h to 80km/h (excluding)))

$$T_1 = \begin{cases} 54.97502 & 8.84 \le Q_1 < 10.43 \\ 147.976 - \dfrac{95.24837}{1 + (\dfrac{Q_1}{12.2655})^{8.67823}} & 10.43 \le Q_1 < 16.99 \\ 146.6 & Q_1 \ge 16.99 \end{cases}$$

where $T_1$- Driver's arousal duration after the fatigue arousal section at the speed 1;
$Q_1$- The stimulus quantity of fatigue arousal section at the speed 1.

② Speed 2 (a specific speed 2 (e.g. 80 km/h) or a speed range 2 (e.g. 80 km/h to 100km/h (excluding)))

$$T_2 = \begin{cases} 64.13252 & 9.42 \le Q_2 < 13.877 \\ 172.6251 - \dfrac{111.1144}{1 + (\dfrac{Q_2}{16.31935})^{8.67823}} & 13.877 \le Q_2 < 22.608 \\ 171.02 & Q_2 \ge 22.608 \end{cases}$$

where $T_2$- Driver's arousal duration after the fatigue arousal section at the speed 2;
$Q_2$- The stimulus quantity of fatigue arousal section at the speed 2.

③ Speed 3 (e. g. a specific speed 3 (e.g. 100 km/h) or a speed range 3 (e.g. 100 km/h to 120km/h))

$$T_3 = \begin{cases} 76.95752 & 17.64 \le Q_3 < 25.987 \\ 207.1461 - \dfrac{133.3347}{1 + (\dfrac{Q_2}{30.5598})^{8.67823}} & 25.987 \le Q_3 < 42.336 \\ 205.22 & Q_3 \ge 42.336 \end{cases}$$

where $T_3$- Driver's arousal duration after the fatigue arousal period at the speed 3;
$Q_3$- The stimulus quantity of fatigue arousal section at the speed 3.

In the embodiment of the disclosure, the mentioned multiple different vehicle speeds may refer to different specific vehicle speeds, such as vehicle speeds (point values) of 40km/h, 60km/h, 80km/h, 100km/h, 120km/h. Or it may also refer to different vehicle speed ranges, such as speed ranges of 40km/h to 60km/h, 60km/h (excluding) to 80km/h, 80km/h (excluding) to 100km/h, 100km/h (excluding) to 120km/h. It may also comprise a combination of specific vehicle speeds (point values) and speed ranges.

In the embodiment of the disclosure, for an arousal duration model for a speed range, it may either use a characterized model for a specific value within the speed range, or the arousal duration model for the speed range may be generated based on samples characterized by the specific values within the speed range. For example, for a model for the aforementioned speed range 1 (60 km/h to 80 km/h), it may either use a characterized model at 60 km/h as the model for the speed range 1, or in another embodiment, the model for the speed range 1 may be generated based on the samples obtained at 60 km/h.

Accordingly, the stimulus time may be determined based on the above arousal duration model with the first plateau segment, the second rising segment, and the third plateau segment, and then the stimulus quantity and the arousal duration may be determined accordingly, such that the length and spacing of the fatigue arousal section is determined.

In some embodiments of the disclosure, the arousal duration model may be generated based on the fifth samples obtained at a reference speed. The reference speed may be, for example, zero, i.e., in a stationary state.

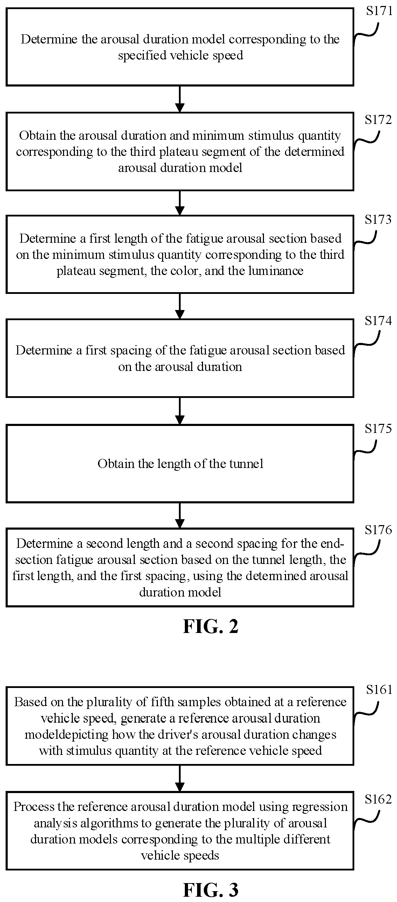

For example, in the embodiment illustrated in FIG. 3, the step S160 may comprise:

S161: based on the plurality of fifth samples obtained at a reference vehicle speed, generating a reference arousal duration model depicting how the driver's arousal duration changes with stimulus quantity at the reference vehicle speed; and

S162: processing the reference arousal duration model using regression analysis algorithms to generate the plurality of arousal duration models corresponding to the multiple different vehicle speeds.

In a preferred embodiment, the fifth samples are obtained from experimental drivers in a stationary state. That is, the reference vehicle speed is preferably zero.

In an embodiment not shown, the fifth samples may be obtained separately at different vehicle speeds, and then the models may be fitted and thus generated separately.

In a further embodiment, the fifth sample may be derived from the data obtained from the same experiment as the other samples, as further described below.

For example, in the embodiment shown in FIG. 2, the step S170 may comprise:

S171: determining the arousal duration model corresponding to the specified vehicle speed;

S172: obtaining the arousal duration and minimum stimulus quantity corresponding to the third plateau segment of the determined arousal duration model;

S173: determining a first length of the fatigue arousal section based on the minimum stimulus quantity corresponding to the third plateau segment, the color, and the luminance; and

S174: determining a first spacing of the fatigue arousal section based on the arousal duration.

In some embodiments of the disclosure, in the step S180, all the fatigue arousal sections may be arranged based on the aforementioned first length and first spacing, meaning that all the fatigue arousal sections in the tunnel, such as in the long tunnel, have the aforementioned first length and the aforementioned first spacing therebetween.. In some embodiments of the disclosure, in the step S180, at least one, such as a portion of, fatigue arousal sections, may be arranged based on the aforementioned first length and first spacing, while other fatigue arousal section's length, spacing or other characteristics may be set according to further preferred embodiments of the disclosure or using empirical data, such that the solution of arranging the at least one or the portion of fatigue arousal sections still represent advantages over the prior art. In the disclosure, the spacing for the fatigue arousal sections is broadly interpreted to comprise the spacing between fatigue arousal sections, the spacing between the fatigue arousal section and the tunnel opening (exit and/or entrance), and/or the spacing between the fatigue arousal section and other functional section.

In a preferred embodiment of the disclosure, the length of the fatigue arousal section and its spacing to the tunnel exit may be specifically provided for the final section of the tunnel, such that the arrangement may fully take into account the user's physiological and psychological consistency throughout the entire tunnel, helping adaptively providing optimized energy saving for each tunnel.

Optionally, as further shown in FIG. 2, the step S170 may further comprise:

S175: obtaining the length of the tunnel; and

S176: based on the tunnel length, the first length, and the first spacing, determining a second length and a second spacing for the end-portion fatigue arousal section using the determined arousal duration model.

Accordingly, in the embodiment comprising the steps S170 to S176 as shown in FIG. 2, the step S180 may comprise: arranging middle-portion fatigue arousal sections in the tunnel's middle section based on the first length and the first spacing, and arranging an end-portion fatigue arousal section in the tunnel's end section based on the second length and second spacing.

In the embodiment of the disclosure, the fatigue arousal section comprise wall washer lights alternating in multiple colors installed on both sides of the road, with the wall washer lights having the specified luminance.

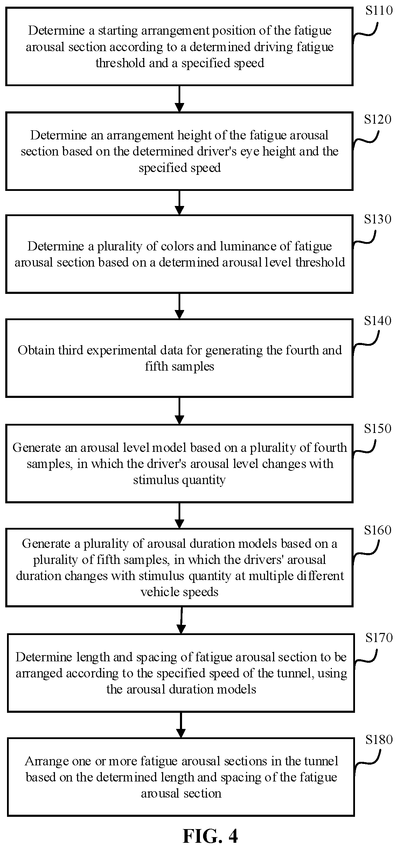

Furthermore, in a preferred embodiment of the disclosure, the method for arranging fatigue arousal sections in tunnels may comprise one or more optional steps (features) in addition to the steps S130, S160, S170, S180 in the aforementioned embodiment shown in FIG. 1. In an embodiment as shown in FIG. 4, the method for arranging fatigue arousal sections in tunnels comprises one or more optional steps. Those skilled in the art will understand that one or more optional steps (features) further illustrated in the embodiment shown in FIG. 4 may be incorporated into the embodiment shown in FIG. 1 either individually or in combination to obtain new embodiments.

In the embodiment of the disclosure, the method for arranging fatigue arousal sections in tunnels may also comprise optional steps (features) to obtain an increased arousal level for constraining the arousal duration model generated in the step S160.

For example, in the embodiment shown in FIG. 4, the method may further comprise an optional step S150:
S150: based on a plurality of fourth samples, generating an arousal level model in which the driver's arousal level changes with stimulus quantity.

In the embodiment of the disclosure, the fourth samples comprise the different drivers' arousal levels at different stimulus quantities with the plurality of colors and luminance.

Accordingly, the step S160 may comprise: constraining the plurality of arousal duration models using a minimum stimulus quantity determined by the arousal level model.

In some embodiments, the minimum stimulus quantity determined by the arousal level model may be used to constrain the range of stimulus quantities in the arousal duration models. For example, the endpoint values q0, q1, and q2 of the aforementioned stimulus quantity range, are constrained by the minimum stimulus quantity. As an example, if the minimum stimulus quantity is greater than the endpoint value q0 obtained from the model obtained through fitting and regression analysis of or direct fitting of the fifth samples, then the minimum stimulus quantity is used as the endpoint value q0. A portion of the first plateau section, second rising section, or third plateau section are constrained by the minimum stimulus quantity, thus for example being partially effective. As an example, if the minimum stimulus quantity is greater than or equal to the endpoint value q1 obtained from the model obtained through fitting and regression analysis of or direct fitting of the fifth samples, obtained by the fifth sample fitting and regression analysis or directly from the model, then only the second rising section (part of) and the third plateau section are effective in the corresponding arousal duration model.

Therefore, the minimum stimulus quantity of constraint determined by the arousal level model can achieve an optimal implementation of effective fatigue arousal section, thereby meeting the physiological and psychological needs of drivers for safer and more comfortable driving in various long tunnels.

In the embodiment of the disclosure, common experimental data may be utilized for generating both the arousal duration model and the arousal level model, providing a foundation with enhanced consistency and scientific validity.

For example, in the embodiment shown in FIG. 4, the method may also comprise an optional step S140:
S140: obtaining third experimental data for generating the fourth and fifth samples.

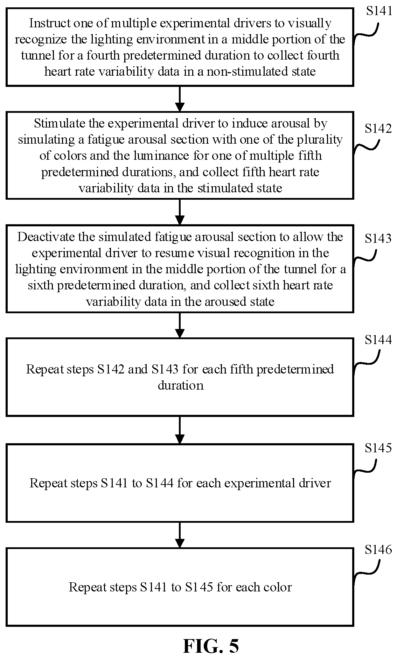

Specifically, in the embodiment shown in FIG. 5, the step S140 may comprise:

S141: instructing one of multiple experimental drivers visually recognize the lighting environment in a middle portion of the tunnel for a fourth predetermined duration to collect fourth heart rate variability data in a non-stimulated state;
S142: stimulating the experimental driver to induce arousal by simulating a fatigue arousal section with one of the plurality of colors and the luminance for one of multiple fifth predetermined durations, and collecting fifth heart rate variability data in the stimulated state;
S143: deactivating the simulated fatigue arousal section to allow the experimental driver to resume visual recognition in the lighting environment in the middle portion of the tunnel for a sixth predetermined duration, and collecting sixth heart rate variability data in the aroused state;
S144: repeating steps s142 and s143 for each fifth predetermined duration;
S145: repeating steps s141 to s144 for each experimental driver; and
S146: repeating steps s141 to s145 for each color.

Those skilled in the art will understand that the cyclic steps described in steps S144, S145, S146 may be implemented in various nested ways.

In the embodiment of the disclosure, the fourth samples are obtained from the fourth and fifth heart rate variability (HRV) data. In the embodiments of the disclosure, the fifth samples are obtained at least from the sixth heart rate variability data.

In a further embodiment, the fourth samples may be for example determined based on a given model of the arousal level versus the heart rate variability data. For example, the model may be used to determine the arousal levels of different drivers under different stimulus durations (and optionally in different colors) based on the fourth and fifth heart rate variability data, and the corresponding stimulus quantities may be determined based on stimulus duration, color, and luminance, thus obtaining the fourth samples.

In a further embodiment, for example, the fifth samples may be determined based on a given model of arousal duration versus heart rate variability data and/or a determined arousal (fatigue) critical HRV value or a determined method. For example, based on the arousal (fatigue) critical heart rate variability value determined through various methods and the sixth heart rate variability data, the time point at which the driver reaches the critical state of arousal (fatigue), i.e., the endpoint of arousal duration, may be determined, such that the arousal duration (period) may be determined. And accordingly, the corresponding stimulus quantity may be determined based on the stimulus duration, the color, and the luminance, such that the fifth samples are obtained. Alternatively, the endpoint of the arousal duration may be determined based on a certain arousal (fatigue) critical heart rate variability value method, for example, by determining when the driver's heart rate variability (fifth heart rate variability data) decreases by a predetermined amount from the heart rate variability at the cessation of stimulation, based on the obtained sixth heart rate variability data. All of these methods fall into the scope of the disclosure.

In a further embodiment, the fifth samples may be obtained independently or exclusively.

In the embodiment of the disclosure, the arousal level may be used to support the selection of color and luminance of the fatigue arousal section. For example, in the embodiment shown in FIG. 4, the step S130 of the method may comprise additional features of determining the plurality of colors and luminance of the fatigue arousal section based on a determined arousal level threshold.

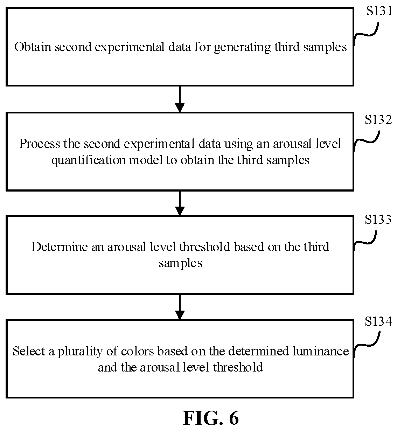

In a further embodiment, as shown in FIG. 6, the step S130 may comprise:

S131: obtaining second experimental data for generating third samples;
S132: processing the second experimental data using an arousal level quantification model to obtain the third samples;
S133: determining an arousal level threshold based on the third samples; and
S134: selecting a plurality of colors based on the determined luminance and the arousal level threshold. In the embodiment of the disclosure, the third samples comprise the arousal levels of different drivers under different colors.

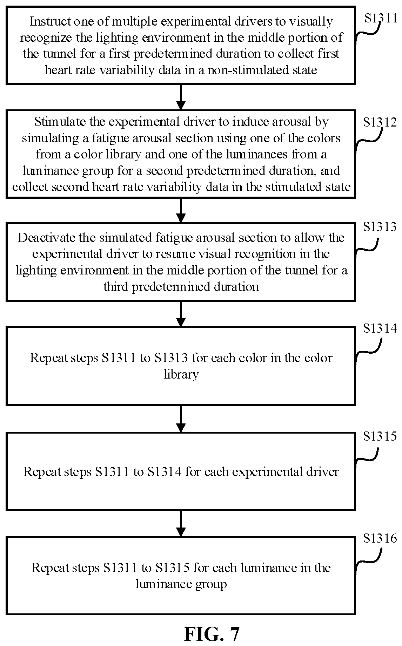

In a further embodiment, as shown in FIG. 7, the step S131 may specifically comprise:

S1311: instructing one of multiple experimental drivers to visually recognize the lighting environment in the middle portion of the tunnel for a first predetermined duration to collect first heart rate variability data in a non-stimulated state;
S1312: stimulating the experimental driver to induce arousal by simulating a fatigue arousal section using one of the colors from a color library and one of the luminance from a luminance group for a second predetermined duration, and collecting second heart rate variability data in the stimulated state;
S1313: deactivating the simulated fatigue arousal section to allow the experimental driver to resume visual recognition in the lighting environment in the middle portion of the tunnel for a third predetermined duration;
S1314: repeating steps s1311 to s1313 for each color in the color library;
S1315: repeating steps s1311 to s1314 for each experimental driver; and
S1316: repeating steps s1311 to s1315 for each luminance in the luminance group.

Similarly, the cyclic steps described in the steps S1314, S1315, S1316 may be implemented in various other nested ways, which falls into the scope of the disclosure.

In the embodiment of the disclosure, the third samples are obtained based on the first and second heart rate variability (HRV) data, and the number of colors in the color library is greater than or equal to the number of the selected plurality of colors.

Similarly, the third samples may be determined based on a given model of arousal level versus heart rate variability data. For example, the model may be used to determine the arousal levels of different drivers under various luminance for a given color at the second predetermined duration based on the first and second heart rate variability data, and correspondingly, determine the corresponding stimulus quantity based on the second predetermined duration, color, luminance, such that the third samples are obtained. Optionally, when deactivating the simulated fatigue arousal section to allow the experimental driver to resume visual recognition in the lighting environment in the middle portion of the tunnel, third heart rate variability data may be collected for further study.

In some embodiments, the arousal quantification model for processing the second experimental data may be shown as following equation.

$$W = \left[ \left( \frac{LF}{HF} \right)_b - \left( \frac{LF}{HF} \right)_a \right] \Big/ \left( \frac{LF}{HF} \right)_a$$

where W -fatigue arousal level;

$$\left(\frac{LF}{HF}\right)_b$$ -HRV value of the driver at 30 seconds after the fatigue arousal section stimulus;

$$\left(\frac{LF}{HF}\right)_a$$ -HRV value of the driver before the fatigue arousal segment stimulus.

In some embodiments, the arousal level LF/HF variability rate index value for a given percentage of drivers (i.e. 85th percentile), denoted as W=2.1, may be used as a representative value indicating that the stimulus quantity of the fatigue arousal section is able to recover the driver to an alert state after arousal. The value may be used in selection of colors for the fatigue arousal section.

In optional embodiments of the disclosure, the luminance determined in the step S130 may be selected based on the road safety requirements as described below and as described above, may be used to determine the plurality of colors of the fatigue arousal section, such as for parameters in the previous experiment. For example, the determined luminance may be taken as the midpoint value of the experimental luminance range, to obtain the experimental luminance group.

In the embodiment of the disclosure, the starting position for arranging the fatigue arousal section may also be optionally determined. The arragement may be based, at least partially, on the determined driving fatigue threshold and the specified vehicle speed of the tunnel. Such an arrangement may optimize the physiological and psychological needs of drivers for safe and comfortable driving in the various long tunnels of varying lengths.

For example, in the embodiment shown in FIG. 4, the method optionally comprises:
S110: determining a starting arrangement position of the fatigue arousal section according to a determined driving fatigue threshold and the specified speed.

In a further embodiment, the step S180 optionally comprises : arranging one or more fatigue arousal sections in the tunnel based on the starting arrangement position, the length and the spacing of the fatigue arousal section.

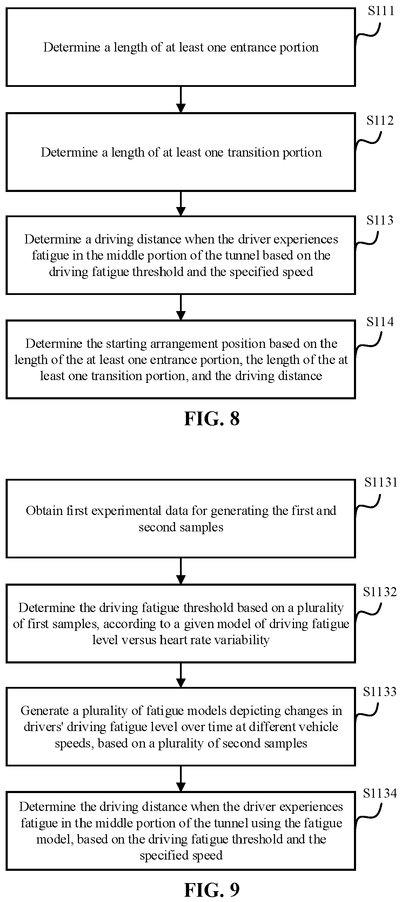

In a further embodiment, as shown in FIG. 8, the step S110 optionally comprises:

S111: determining a length of at least one entrance portion;
S112: determining a length of at least one transition portion;
S113: determining a driving distance when the driver experiences fatigue in the middle portion of the tunnel based on the driving fatigue threshold and the specified speed; and
S114: determining the starting arrangement position based on the length of the at least one entrance portion, the length of the at least one transition portion, and the driving distance.

In the embodiment of the disclosure, the length of the entrance portion may be determined according to a given model, empirical formula, or a safety constraint. Optionally, the determination of the length of the entrance portion may be determined in any known or novel manner.

In the embodiment of the disclosure, the determination of the length of the transition portion may be determined according to a given model, an empirical formula, or a safety constraint. Optionally, the determination of the length of the transition portion may be determined in any known or novel manner, such as in a manner taught in embodiments of the disclosure.

In embodiments of the disclosure, the entrance portion and middle portion have their conventional meanings in this field. In the embodiments of the disclosure, the transition portion refers to a road section between the entrance portion and the middle portion.

In a further embodiment, as shown in FIG. 9, the step S113 optionally comprises:

S1132: determining the driving fatigue threshold based on a plurality of first samples, according to a given model of driving fatigue level versus heart rate variability;
S1133: generating a plurality of fatigue level models depicting changes in drivers' driving fatigue level over time at different vehicle speeds, based on a plurality of second samples; and
S1134: determining the driving distance when the driver experiences fatigue in the middle portion of the tunnel using the fatigue model, based on the driving fatigue threshold and the specified speed.

In the embodiment of the disclosure, the first samples comprise heart rate variability (HRV) of different drivers driving in a calm state and when feeling fatigued in the tunnel environment.

In the embodiment of the disclosure, the second samples comprise driving fatigue levels of different drivers at different times when driving in the tunnel environment at different vehicle speeds.

Similarly, the first or second sample may be determined based on a given model of driving fatigue levels versus heart rate variability data. For example, using the model, the first samples may be determined by determining the heart rate variability (difference) of drivers when they feel fatigued after entering the middle portion from the tunnel entrance, based on the heart rate variabilities in a calm state and when feeling fatigued while driving in the tunnel environment. For example, using the model, the second samples may be determined based on the heart rate variability while driving in the tunnel environment.

For example, in the embodiment illustrated in FIG. 9, the step S113 may also optionally comprise:
S1131: obtaining first experimental data for generating the first and second samples.

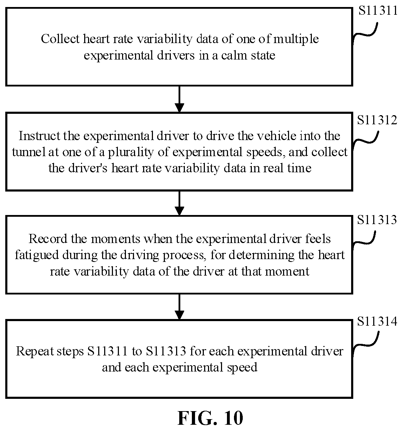

In the embodiment illustrated in FIG. 10, the step S113 may optionally specifically comprise:

S11311: collecting heart rate variability data of one of multiple experimental drivers in a calm state;
S11312: instructing the experimental driver to drive the vehicle into the tunnel at one of a plurality of experimental speeds, and collecting the driver's heart rate variability data in real time;
S11313: recording the moment when the experimental driver feels fatigued during the driving process, for determining the heart rate variability data of the driver at that moment; and
S11314: repeating steps S11311 to S11313 for each experimental driver and each experimental speed.

Optionally, the model of driving fatigue versus heart rate variability is as follows:

$$A_{ij} = \left[ \left( \frac{LF}{HF} \right)_{ij} - \left( \frac{LF}{HF} \right)_i \right] \Big/ \left( \frac{LF}{HF} \right)_i$$

where $A_{ij}$ - Driving fatigue of $i^{th}$ driver i at driving time j in the tunnel;

$\left( \dfrac{LF}{HF} \right)_{ij}$ - the HRV value of $i^{th}$ driver at the $j^{th}$ moment while driving in the tunnel;

$\left( \dfrac{LF}{HF} \right)_i$ - the initial HRV value of $i^{th}$ driver in calm state, represented by the mode.

In some embodiments, the fatigue driving threshold is determined using the model of driving fatigue versus heart rate variability for a given proportion of driver (person) at the moments of fatigue.

In some embodiments, the driving fatigue level model is shown as follows:

① Speed 1 (specific speed 1 (e. g. 60 km/h) or speed range 1 (e. g. 60 km/h to 80km/h (excluding)))

$$A = 1.80869 + \frac{163.00287}{104.6879\sqrt{\frac{\pi}{2}}} * e^{-2\left( \frac{t-146.7103}{104.6879} \right)^2}$$

② Speed 2 (specific speed 2 (e.g. 80 km/h) or speed range 2 (e.g. 80 km/h to 100km/h (excluding)))

$$A = 1.91254 + \frac{181.81534}{124.46489\sqrt{\frac{\pi}{2}}} * e^{-2\left( \frac{t-174.20238}{124.46489} \right)^2}$$

③ Speed 3 (specific speed 3 (e.g. 100 km/h) or speed range 2 (e.g. 100 km/h to 120km/h))

$$A = 2.01221 + \frac{192.91589}{146.19899\sqrt{\frac{\pi}{2}}} * e^{-2\left( \frac{t-226.83989}{146.19899} \right)^2}$$

where A - the driving fatigue of drivers in a long tunnel;

t - Driving time (in seconds) in the middle portion of the long tunnel.

In some embodiments, the time at which a driver becomes fatigued at the specified speed may be determined under the determined fatigue driving threshold and the specified speed, and the driving distance is thus determined.

In the embodiment of the disclosure, the setting height of the fatigue arousal section can also be selectively determined, and this setting height may be based at least partially on the determined driver's eye level and the specified vehicle speed. Such a setting can optimize the physiological and psychological needs of drivers for safe and comfortable driving in tunnels of varying lengths.

For example, in the embodiment shown in FIG. 4, the method may optionally comprise:

S120: determining an arrangement height of the fatigue arousal section based on the determined driver's eye height and the specified speed. Accordingly, the step S180 may comprise: arranging the one or more fatigue arousal sections in the tunnel based on the arrangement height, the length, and the spacing of the fatigue arousal section.

In a preferred embodiment of the disclosure, the step S180 may comprise: arranging one or more fatigue arousal sections in the tunnel based on the determined starting arrangement position, arrangement height, length, and spacing of the fatigue arousal section.

In the embodiment of the disclosure, a device for arranging a fatigue arousal section in a tunnel is also provided, comprising: a first determination unit, configured to determine a plurality of colors and luminance for the fatigue arousal section; a generation unit, configured to generate a plurality of arousal duration models based on a plurality of fifth samples in which the drivers' arousal duration changes with stimulus quantity at a plurality of different vehicle speeds, the fifth samples comprising arousal durations of different drivers under different stimulus quantities at the multiple colors and luminance; a second determination unit, configured to determine a length and a spacing of the fatigue arousal section to be arranged according to the specified speed of the tunnel using the arousal duration models; an arrangement unit, configured to arrange one or more fatigue arousal sections in the tunnel based on the determined length and spacing of the fatigue arousal section.

With the teaching of the disclosure, the features of method embodiments may be combined with the device embodiments in a non-contradictory manner to obtain new embodiments, and the features of device embodiments may also be combined with method embodiments in a non-contradictory manner to obtain new embodiments, all of which fall into the scope of the disclosure.

In an embodiment of the disclosure, a road tunnel is provided, which comprises a fatigue arousal section arranged according to the arrangement method of any one of the embodiments of the disclosure.

In order to further describe the various embodiments of the disclosure, and to show how the fatigue arousal section is able to meet the driver safety requirements during use, the arrangement position, height, color type, luminance and length of the fatigue arousal section design are described in the form of the specific embodiment, and corresponding experiments and analysis are provided. Here, the features of the specific embodiments below can be incorporated into the respective embodiments described above individually or in combination in a non-contradictory manner to obtain new embodiments. In particular, the formula of the specific embodiments below may be combined with the various embodiments described above to obtain new embodiments, which fall into the scope of the disclosure.

Arrangement Position of Fatigue Arousal Section

As an explanation rather than a limitation, when driving at different speeds in the middle portion of a long tunnel, the difficulty of driving varies. The higher the speed is, the more difficult the driving task will face, and the less likely drivers are to reach a state of driving fatigue. Drivers reach a low-load driving fatigue state at different times depending on their operating speed, and thus have different needs for the arrangement position of fatigue arousal sections. In embodiments of the disclosure, the driver's fatigue level may be quantified using physiological and psychological indicators. Optionally, the driver's driving workload index may be determined based on the driver's driving behavior characteristics and the formation pattern of driver fatigue to represent the driver's driving fatigue. More specifically, the driver's driving workload is determined by the relationship between speed and the driver's heart rate variability index.

Here, the following experimental examples are proposed, which may, for example, obtain the first experimental data for determining the starting arrangement position of the fatigue arousal section, to be used for generating the first and second samples.

1. Experimental Setup

Using a combination of quantitative physiological and psychological HRV (Heart Rate Variability) indicators and subjective qualitative evaluation indicators, the real-time physiological and psychological parameter values of test drivers were measured when driving in long tunnels under free-flow conditions at speeds of 60km/h, 80km/h, and 100km/h. The driving fatigue threshold for drivers in the middle portion of long tunnels was determined; models of how drivers' fatigue levels change with driving time at different operating speeds were analyzed, and finally, the moment when driving fatigue occurs in the middle portion of the long tunnel and the arrangement position of fatigue arousal section were determined.

2. Experimental Subjects

According to the experimental purpose, a random sampling method was used to select 24 healthy drivers for the experiment. The test drivers were required to have no alcohol consumption or medication before the experiment, to be well-rested and have normal reactions. The experimental drivers' uncorrected vision in both eyes had to be 4.9 or above on the vision chart, with no eye diseases such as color blindness or color weakness. Since small passenger car drivers have a lower eye height than large truck drivers and a less open field of vision, it is more difficult to visually recognize target objects under the same lighting environment. To ensure the validity of the experimental data, following the most unfavorable principle, typical small passenger cars were selected as the experimental vehicle type.

3. Experimental Equipment

To measure different luminance and the wavelength values of different colors on the side walls of simulated fatigue arousal section in long tunnel middle portion, a CS-150 single-lens reflex spot luminance meter was used, which can measure light sources and surface luminance. The luminance measurement range is 0.001 to 299,900 cd/m$^2$。

To collect the drivers' ECG indicators during the experiment, a KF2 multi-parameter physiological detector was used, equipped with data analysis software to simplify the data processing.

To record the drivers' gaze characteristics during the experiment and assist in recording abnormal events that cause changes in the drivers' physiological and psychological indicators, an iViewX 1.05 build 49 HED dynamic eye tracker was used to record information such as the drivers' fixation points.

To collect the operating speed and driving distance of vehicles in the long tunnel, and to mark characteristic positions within the long tunnel, a non-contact multifunctional speed meter was used. The instrument has a sampling frequency of 60Hz, a speed measurement range of 0~250.00km/h with a minimum resolution of 0.01km/h, and a distance measurement range of 0~99999.999m with a minimum resolution of 1mm.

4. Experimental Procedure

To eliminate the influence of time and abnormal events, each driver performed experiments at the same speed in the morning from 9:00-11:00 and in the afternoon from 3:00-5:00. The specific experimental steps are described as follows: (1) Each test driver is equipped with a dynamic physiological and psychological detector and an eye tracker, and install a non-contact multifunctional speed meter in the vehicle. To ensure the validity of the experimental data, the time of each instrument must be accurately calibrated before each experiment to keep the time of all experimental detection instruments consistent.

(2) One experimenter sits in the passenger seat to record the driver's fatigue status and any abnormal conditions during vehicle operation at any time.

(3) The test driver fills out the pre-driving fatigue level according to the driving fatigue level survey questionnaire in Table 1. The experimenter collects the driver's ECG data for 3 minutes while the driver is in a calm state sitting in the driver's seat before driving.

(4) The driver starts driving the vehicle 200m before the entrance of the long tunnel at operating speeds of 60km/h, 80km/h, and 100km/h respectively. When the driver feels they are in a level 3 fatigue state during driving, they inform the experimenter, who then promptly marks using the non-contact multifunctional speed meter.

(5) Repeat steps (1) to (4) until the experiment is completed.

**Table 1: Subjective Evaluation Scale of Driving Fatigue**

| Driving fatigue level | Driving fatigue | Physiological status description |
|---|---|---|
| 1 | Clear-headed | Fully alert, arousal, and capable of focused concentration. |
| 2 | General sober | Relaxed and responsive but not entirely sober. |
| **3** | **Slightly fatigued** | **Experiencing chest tightness, and slightly hazy or unfocused thoughts.** |
| 4 | Fatigued | Feeling significantly depressed, with vague thoughts, struggling to remain arousal. |

(continued)

| Driving fatigue level | Driving fatigue | Physiological status description |
|---|---|---|
| 5 | Fatigued | Drowsy, dizzy, struggling against sleep, and a strong desire to fall asleep. |

5. Generation of Driving Fatigue Level Model

In some embodiments, the heart rate variability (HRV) data is represented as LF (low frequency)/HF (high frequency) data.

The parameters collected by the physiological and psychological parameter detector, eye tracker, and non-contact multifunctional speed meter at different operating speeds were matched. The invalid data affected by abnormal events is excluded based on the driver's gaze point information collected by the eye tracker. The average value of two experimental data sets for each driver was taken according to the corresponding same experimental duration. A total of 16,728 LF/HF data samples from the drivers' physiological and psychological experiment array are obtained.

To exclude the influence of different heart rate differences among different drivers, and to obtain the uniform driving fatigue threshold applicable to drivers in the long tunnel, the calculated LF/HF value are subtracted by the mode of the LF/HF initial values in the calm state of the test drivers before the experiment in the section. The result is then divided by the mode of the LF/HF initial values to obtain a LF/HF change rate for each driver which is defined as the driving fatigue degree of the driver in special tunnel ($A_{ij}$). The quantitative model is shown in Equation (1).

$$A_{ij} = \left[ \left( \frac{LF}{HF} \right)_{ij} - \left( \frac{LF}{HF} \right)_i \right] / \left( \frac{LF}{HF} \right)_i \qquad (1)$$

where $A_{ij}$- driving fatigue of $i^{th}$ driver at $j^{th}$ moment while driving in the tunnel;

$\left( \frac{LF}{HF} \right)_{ij}$ - the HRV value of $i^{th}$ driver at $j^{th}$ moment while driving in the tunnel;

$\left( \frac{LF}{HF} \right)_i$ - the initial HRV value of $i^{th}$ driver in calm state, represented by the mode.

By analyzing the driving fatigue degree of 24 drivers at the moment of driving fatigue after entering the middle section of long tunnels under different operating speed conditions, the distribution pattern is shown in Figure 2. Since there was no significant difference in the driving fatigue degree at the moment of driving fatigue for the 24 drivers under different operating speed conditions, according to the requirement in traffic engineering that the sample size should meet the driving needs of more than 85% of drivers, the fatigue threshold for setting fatigue arousal sections that can satisfy more than 85% of drivers reaching driving fatigue (i.e., feeling fatigued as mentioned in the previous embodiment) was determined to be 3.02. When drivers enter the middle section of the tunnel and drive for a period of time, driving fatigue begins to occur when their LF/HF value increases to 3.02 times (A=3.02) their LF/HF value in the non-driving state.

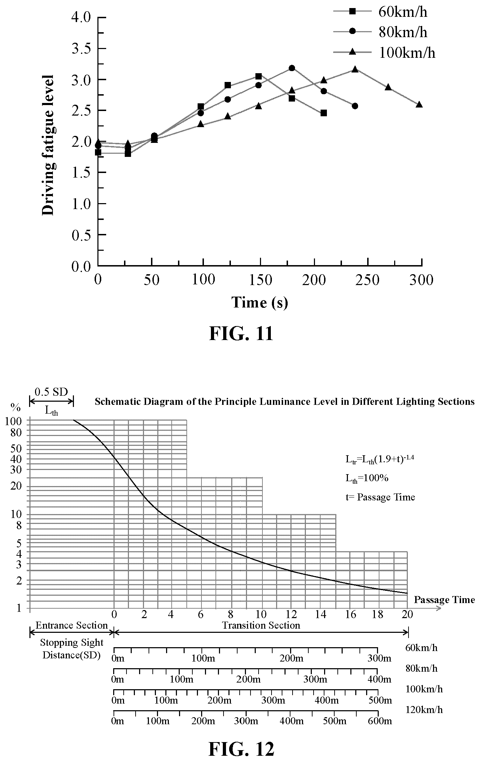

The average value of driving fatigue of 24 drivers after entering the middle portion of the tunnel at different operating speeds was taken as the characteristic value of driving fatigue of all drivers, and the variation pattern of the average driving fatigue degree of 24 drivers under different running speed conditions is shown in FIG. 11.

By fitting the driving fatigue levels under different operating speed conditions, the models of how drivers' fatigue levels change with time when driving in the middle section of long tunnels at different operating speeds are shown in equations (2), (3), and (4) respectively.

① Speed of 60km/h

$$A = 1.80869 + \frac{163.00287}{104.6879\sqrt{\frac{\pi}{2}}} * e^{-2\left( \frac{t-146.7103}{104.6879} \right)^2} \qquad (2)$$

② Speed of 80km/h

$$A = 1.91254 + \frac{181.81534}{124.46489\sqrt{\dfrac{\pi}{2}}} * e^{-2\left(\frac{t-174.20238}{124.46489}\right)^2} \qquad (3)$$

③ Speed of 100km/h

$$A = 2.01221 + \frac{192.91589}{146.19899\sqrt{\dfrac{\pi}{2}}} * e^{-2\left(\frac{t-226.83989}{146.19899}\right)^2} \qquad (4)$$

where A - the driving fatigue level of drivers in the long tunnel;

t -driver's driving time in the middle portion of the long tunnel (in seconds).

The driving fatigue threshold A =3.02 in the mediate section of the long tunnel is substituted into the equations (2), (3) and (4). The driver's fatigue time when driving at speeds of 60 km/h, 80 km/h, and 100 km/h is calculated as t = 146.60s, 171.02s, and 205.22s, respectively. That is, the driver starts to experience driving fatigue when driving about 146.6×60/3.6=2.44km, 171.02×80/3.6=3.8km and 205.22×100/3.6=5.7km into the middle portion of the long tunnel at speeds of 60 km/h, 80 km/h, and 100 km/h, respectively. The positions are the starting positions for arranging the fatigue arousal sections.

The driving distance of the driver experiences fatigue in the middle portion under different operating speed conditions, plus the sum of the length of the tunnel entrance portion(s) and the transition portion(s) to obtain the position of the first driving fatigue arousal section from the entrance of the tunnel in the long tunnel, which may be expressed as shown in equation (5).

$$P = D_f + (D_{th1} + D_{th2}) + (D_{tr1} + D_{tr2} + D_{tr3}) \qquad (5)$$

where P -- distance of the starting position of the fatigue arousal section from the tunnel entrance in the long tunnel;

$D_f$- driving distance when drivers experience fatigue in the middle portion of the long tunnel;

$D_{th\,1}$- length of the long tunnel entrance portion 1;

$D_{th\,2}$- length of the long tunnel entrance portion 2;

$D_{tr\,1}$- length transition portion 1;

$D_{tr\,2}$- length transition portion 2;

$D_{tr\,3}$-length transition portion 3.

In a specific embodiment, the length of a long tunnel entrance portion(s) (such as entrance portion 1 and entrance portion 2) may be adjusted as required.

In a specific embodiment, the transition portion(s) between the tunnel entrance portion(s) and the middle portion of the tunnel may be set to achieve a transition in lighting conditions from outside to inside the tunnel according to the International Lighting Committee (CIE) adaptation luminance curve. In the transition section area, three transition lighting portion(s), namely transition portion(s) I, II and III, are set up with a luminance ratio of 3:1, as shown in FIG. 12, which thus help create a safe and comfortable transition to the driver's visual environment to ensure the safety of operation.

Accordingly, in some embodiments of the present application, the first and second entrance portions and multiple transition portions, preferably three, may be set. The multiple, preferably three, transition portions are set to reduce the luminance from the second entrance section to the middle section in an arithmetic progression.

Arrangement Height of Fatigue Arousal Sections

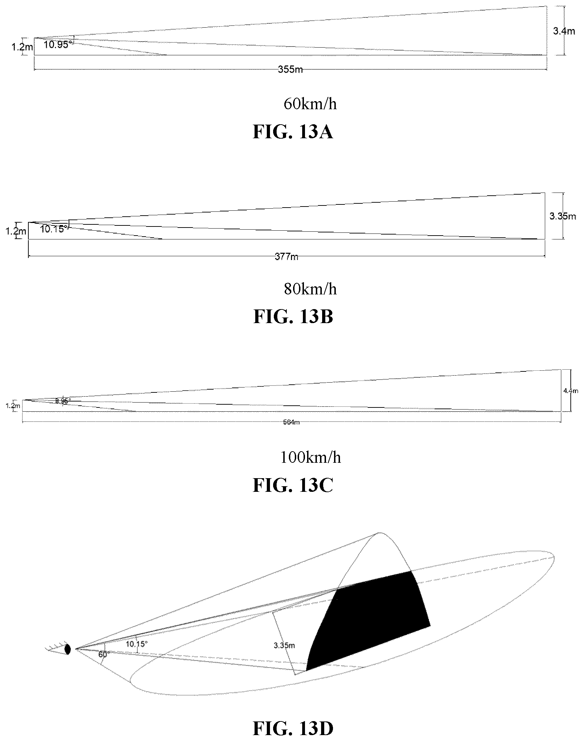

As an explanation rather than a limitation, when drivers are driving in the middle portion of the long tunnel, their gaze is focused on the road surface in front of the driving lane to observe whether the road meets safe driving conditions. Secondly, they perceive information on the side walls and the tunnel ceiling within their dynamic field of view. Since small passenger car drivers have a lower eye height than large truck drivers, their field of view is relatively smaller. In this context, according to the traffic safety facilities set to meet the most unfavorable principle, the setting is based on the passenger car driver's field of vision. Assuming that the eye height of small passenger car drivers is 1.2m, and the vehicle speeds are 60km/h, 80km/h, and 100km/h respectively, it can be detected that the maximum angle range of the drivers' dynamic field of view is 10.95°, 10.15°, and 8.95° respectively. According to the relationship between the driver's dynamic gaze point and the vehicle speed, it may be deduced that when the vehicle speeds are 60 km/h, 80 km/h and 100km/h, the visual heights of the driver on the side wall of the mediate section of the long tunnel are 3.4m, 3.35m and 4.4m respectively that is shown in FIG. 13A, 13B and 13C. FIG. 13D schematically shows the area of potential visual stimulation and side wall height when small passenger car drivers are traveling at 80km/h.

<u>Selection of Colors and Luminance for Fatigue Arousal Section</u>

As an explanation rather than a limitation, the embodiments of the disclosure also focus on considering the stimulus intensity ranking of different color types and the correlation between luminance and drivers' fatigue arousal level.

Here, the following experimental implementation example is proposed, which may be used to determine the colors and luminance adopted for fatigue arousal sections. The experiment may be used, for example, to obtain the aforementioned second experimental data for generating the third samples.

1. Experimental Setup

Using a combination of drivers' subjective evaluation and quantitative research on drivers' physiological and psychological indicators, in a simulated environment of the middle portion of the long tunnel, the subjective evaluation and changes in physiological and psychological indicators of test drivers before and after arousal under different color and luminance conditions were collected. The stimulus intensity patterns of different color types and different luminance conditions were analyzed to obtain color types and luminance that provide higher stimulus intensity while meeting safe driving requirements.

2. Experimental Subjects

According to the purpose of the experiment, a random sampling method was used to select 12 healthy drivers for the static visual recognition experiment. The test drivers were required to have no alcohol consumption or medication before the experiment, to be well-rested and have normal reactions. The test drivers' uncorrected vision in both eyes had to be 4.9 or above on the vision chart, with no eye diseases such as color blindness or color weakness. Since small passenger car drivers have a lower eye height than large trucks and a less open field of vision, it is more difficult to visually recognize target objects under the same lighting environment. To ensure the validity of the experimental data, following the most unfavorable principle, typical small passenger cars were selected as the experimental vehicle type.

3. Selection of Experimental Parameters

Considering the light source characteristics of most tunnel engineering lamps, drivers' light source needs for safe and comfortable driving, and engineering economics, the color temperature of the light source in the simulated middle portion lighting was chosen as 4000K, with a color rendering index of 70, and a road surface luminance of 1 cd/m$^2$. To analyze the driver's requirement for color type and luminance in the driving fatigue arousal section, seven monochrome lights, including red, orange, yellow, green, cyan, blue and purple (all with 100% color saturation), are selected, with side wall luminance values of 0.5 cd/m$^2$, 1cd/m$^2$, 1.5cd/m$^2$ and 2 cd/m$^2$ respectively, resulting in a total of 28 different visual stimulus intensity combinations for the experiment.

4. Experimental Site

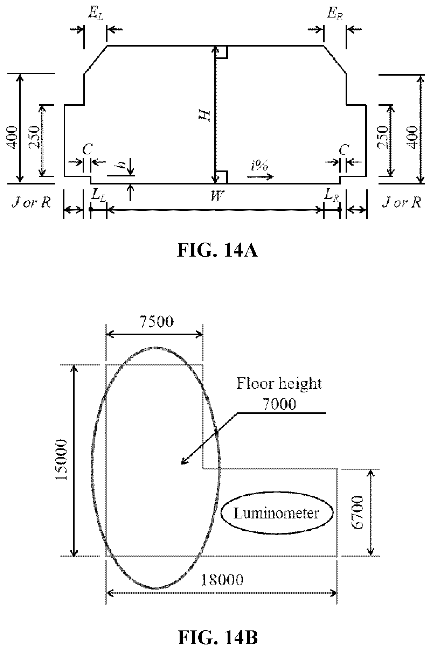

To achieve the experimental objectives and eliminate the influence of other environmental factors, the experiment is carried out in the optical laboratory of a lighting fixture manufacturer. In the embodiment of the disclosure, the standard cross-sectional dimensions of the middle portion of the one-way two-lane expressway tunnel in China is shown in FIG. 14A: the cross section (left maintenance lane 0.75m + left lane width 0.5m + driving lane 3.75m×2 + right lane width 0.75m + right maintenance lane 0.75m) is 10.25m. Accordingly, the experimental field size is 15m×7.5m×7m (see FIG. 14B). In this example, the experiment only simulates the left half of the single tunnel (left maintenance lane 0.75m + left lane width 0.5m + driving lane 3.75m=5m).

5. Experimental equipment

The color luminometer, multi-parameter physiological detector and eye tracker required for the experiment may be the same as those used in the aforementioned experiment.

To set up different visual stimulus intensity fatigue arousal section environments with different color and luminance combinations, RGB three-primary color full-color wall washer lights with a light distribution angle of 30° were selected, which may achieve continuous adjustment of any color wavelength and luminance, as shown in Figure 7. One row of LED wall washer lights, 5m in length, was continuously placed on the inspection road in the simulated middle portion of the tunnel. Based on the calculation of the driver's color recognition visual range, the lights were arranged forwardly in a distance of 4.72 meters in the driving direction (ensuring that the illumination range of the wall washer lights is within the driver's color recognition visual area), without breakpoints, dark area joints, and evenly washing the wall surface.

In this experiment, a simulated tunnel scenario was used, but in various embodiments of the disclosure, for example, in the sub-steps of the aforementioned step S131, the middle portion of the tunnel may be a simulated scenario or an actual scenario, which falls within the scope of the invention.

6. Experimental procedure

The experiment comprises the measurement of physiological and psychological indicators. Optionally, the experiment may also comprise subjective evaluation.

1) Subjective evaluation

After completing all different color fatigue arousal tests under each group of side wall luminance conditions, the test drivers scored each fatigue arousal section color according to their subjective feeling of the degree of fatigue arousal for different fatigue arousal section colors, ranking them by stimulus intensity. They filled out a subjective scoring table, with the color stimulus intensity subjective scoring table shown in Table 2. The scoring was based on the degree of fatigue arousal for different fatigue arousal section colors, with the color having the lowest stimulus intensity scoring 1 and the color with the highest stimulus intensity scoring 7.

**Table 2: Subjective Scoring Table of Color Stimulus Intensity**

| | Experimental driver number | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| **Red** | | | | | | | | | | | | |
| **Orange** | | | | | | | | | | | | |
| **Yellow** | | | | | | | | | | | | |
| **Green** | | | | | | | | | | | | |
| **Cyan** | | | | | | | | | | | | |
| **Blue** | | | | | | | | | | | | |
| **Purple** | | | | | | | | | | | | |

2) Quantitative Measurement of Physiological and Psychological Indicators

Before the experiment began, the test drivers were fully familiarized with the experimental process and requirements, and the order of color stimuli for the fatigue arousal sections was randomly selected. The specific implementation steps for the quantitative measurement of physiological and psychological indicators are as follows:

(1) The lighting fixtures in the simulated tunnel are adjusted to ensure that the road surface, side wall luminance, and road surface uniformity meet the requirements of the experimental scheme.

(2) The driver wears the experimental equipment, becomes fully familiar with the experimental process and requirements, and randomly selects the order of color stimuli for the fatigue arousal section (taking the first group of experimental colors as an example).

(3) The test driver visually recognizes in the lighting environment in the simulated middle portion of the tunnel for 3 minutes with the wall washer lights turned off, simulating the driving state in the tunnel middle section environment. Then it may turn on the wall washer lights and adjust to red fatigue arousal section, with the side wall luminance set to 0.5 cd/m$^2$.

(4) The color luminance meter is used to measure the tunnel side wall luminance and wavelength, with the current color stimulus arousal of the wall washer lights maintained for 10s.

(5) The wall washer lights are turned off, and the driver resumes visual recognition in the simulated middle portion tunnel lighting environment for 3min. The wall washer lights are then switched to the next color (orange) with the luminance kept unchanged.

(6) Steps (4) and (5) are repeated until all color fatigue arousal section stimulus arousal experiments under the luminance of 0.5 cd/m$^2$ are completed.

(7) The next test driver is tested, with steps (2) to (6) repeated until all 12 test drivers complete the stimulus arousal experiments for all color fatigue arousal sections under the side wall luminance of 0.5 cd/m$^2$.

(8) The next group of fatigue arousal section side wall luminance is set, with steps (2) to (7) repeated until all 12 test drivers complete all fatigue arousal section color stimulus arousal experiments under 4 groups of luminance.

7. Analysis of Color Type and Luminance Requirements for Fatigue Arousal Section

① Subjective Evaluation Characteristic Patterns

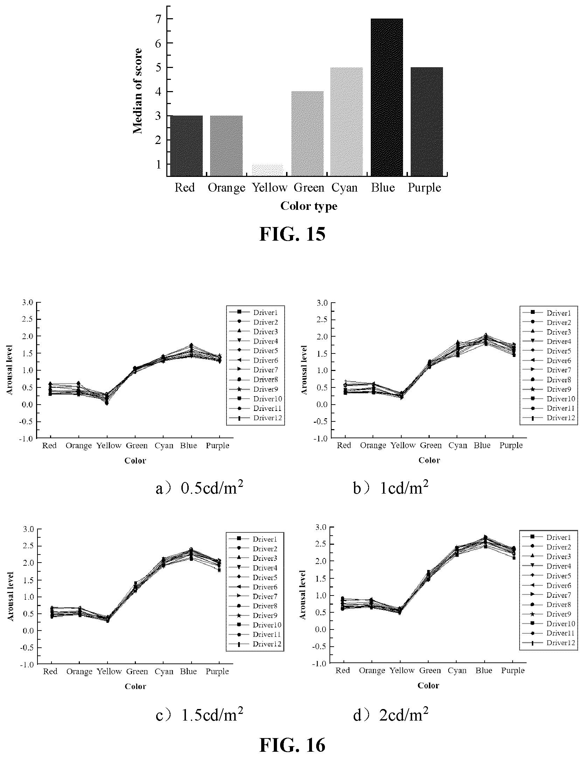

The subjective scoring data of 12 drivers under 7 monochromatic lights and 4 luminance levels were processed. The median of the subjective scores of 12 drivers for each luminance group was used to determine the stimulus intensity ranking of 7 fatigue arousal section colors. The subjective evaluation results are shown in Figure 15. Under the same fatigue arousal section side wall luminance conditions, blue is the color with the highest stimulus intensity, followed by cyan and purple, while yellow has the lowest stimulus intensity. The subjective evaluation ranking of color arousal stimulus intensity is: blue, cyan, purple, green, red, orange, yellow.

② Quantitative Analysis of Physiological and Psychological Indicators

The physiological and psychological indicator data for each driver during each stimulus intensity arousal experimental period were divided, obtaining 28 groups of data for each driver under 7 colors and 4 luminance levels of arousal effects. Each group of data contained corresponding data from the ordinary lighting period in the middle portion and different stimulus intensity arousal periods. The parameters collected by the physiological and psychological parameter detector under different stimulus intensity combinations were processed according to time to obtain LF/HF data for each driver in each experimental period. Based on the driver's gaze point information recorded by the eye tracker, invalid and abnormal data were excluded, obtaining a total of 62,840 data samples.

To eliminate individual differences among drivers, the change rate of the LF/HF 30s after the fatigue arousal section stimulus relative to the LF/HF before the stimulus arousal was taken as the evaluation index of drivers' arousal degree under different stimuli, to eliminate the influence of differences among different drivers themselves. To better quantify the degree of fatigue arousal for drivers under different stimulus quantities of fatigue arousal sections, a driver arousal degree quantification model was established as shown in equation (6).

$$W = \left[\left(\frac{LF}{HF}\right)_b - \left(\frac{LF}{HF}\right)_a\right] \bigg/ \left(\frac{LF}{HF}\right)_a \qquad (6)$$

where W -Driving fatigue arousal degree;

$\left(\frac{LF}{HF}\right)_b$ -HRV value of the driver 30s after the fatigue arousal section stimulus;

$\left(\frac{LF}{HF}\right)_a$ -HRV values of the driver before the fatigue arousal section stimulus.

A. Colors of Fatigue Arousal Sections

In the embodiment, heart rate variability (HRV) is similarly based on LF/HF values.

Under the same luminance conditions, the drivers' arousal degrees after stimulation by different color fatigue arousal sections are shown in Figure 16. As an explanation rather than a limitation, under different fatigue arousal section side wall luminance environments, the drivers' arousal degrees after stimulation by different fatigue arousal section colors show consistent patterns. The ranking of fatigue arousal section color stimulus intensity based on the quantified results of the LF/HF index is basically consistent with the drivers' subjective evaluation results.

The LF/HF change rate index value for 85th percentile drivers, i.e., W=2.1, was used as a representative value indicating that the stimulus quantity of the fatigue arousal section is able to recover the driver to an arousal state after arousal. In this embodiment, colors that result in a driver arousal degree greater than 2.1 under a 10s stimulus time are suitable for being selected, such as blue, purple, and cyan, as the best main colors for the fatigue arousal section.

B. Luminance of Fatigue Arousal Sections

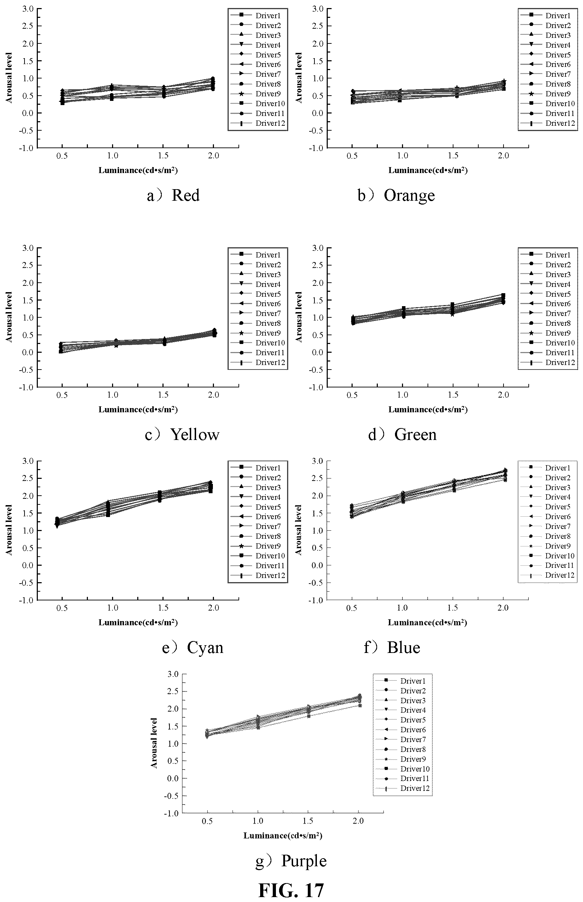

Under different color conditions of fatigue arousal section, the degrees of driver arousal after different fatigue arousal sections are shown in FIG. 17.

In FIG. 17, it can be seen that under the same arousal color conditions, the drivers' arousal degree shows an increasing trend as the side wall luminance increases. For red, orange, yellow, and green fatigue arousal section stimuli, it increases as the luminance increases, but the rate of increase in drivers' LF/HF therefor is lower than for cyan, blue, and purple colors. The luminance settings of fatigue arousal sections should be as high as possible while ensuring safe driving for drivers.

In addition, based on the CIE regulations, in the bright-dark luminance transition inside the tunnel, the luminance of the bright environment should not exceed three times the luminance of the dark environment. In other words, the luminance of the side wall of the fatigue arousal section in the tunnel should not exceed three times the luminance of the normal environmental side wall of the mediate section. As an explanation rather than a limitation, the highest luminance of the mediate section in the long tunnel can be selected according to different design speeds specified in the Highway Tunnel Lighting Design Guidelines (JTG D70/2-01-2014), as shown in Table 3.

**Table 3: Maximum Luminance of Side Wall in the Fatigue Arousal Section at Different Design Speeds**

| Design speed (km/h) | Pavement luminance of mediate section (cd/m$^2$) | Luminance of side wall of mediate section (cd/m$^2$) | Maximum luminance of side wall in the fatigue arousal section (cd/m$^2$) |
|---|---|---|---|
| 100 | 3.0 | 1.8 | 5.4 |
| 80 | 1.5 | 0.9 | 2.7 |
| 60 | 1.0 | 0.6 | 1.8 |

Note: This study was conducted under free-flow conditions, such that the road surface luminance values in the table are selected for low traffic volume indicators.

Length Setting of Fatigue Arousal Sections

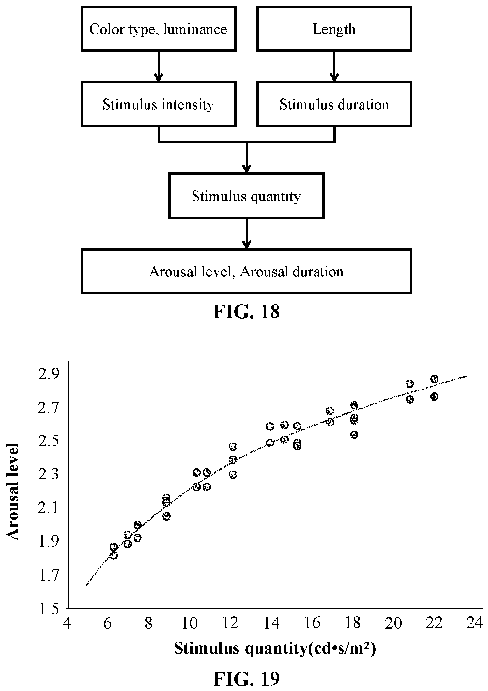

As an explanation rather than a limitation, when drivers are driving in the middle portion of the long tunnel, the set length of fatigue arousal section determines the duration of the fatigue arousal section stimulus. In the embodiments of the disclosure, the stimulus duration of fatigue arousal section and the visual stimulus intensity jointly determine the visual stimulus quantity. The stimulus quantity determines the driver's degree of arousal from driving fatigue and the duration of arousal. To obtain the set length of fatigue arousal section, the required stimulus quantity and stimulus duration for arousing drivers will be studied. In a specific embodiment, the correlation between various factors to be considered for length setting of fatigue arousal sections is shown in Figure 18.

In a specific embodiment, two indicators - the driver's arousal degree and arousal duration - are used to establish correlations with stimulus quantity to determine the stimulus quantity requirements of fatigue arousal sections.

In a specific embodiment, the correlations between visual stimulus quantity and arousal degree and arousal duration are assumed to be as shown in equations (7) and (8). The magnitude of the fatigue arousal section stimulus quantity is the product of stimulus intensity and stimulus duration, as shown in equation (9).

$$W=f_1（Q） \qquad\qquad （7）$$

$$T=f_2（Q） \qquad\qquad （8）$$

where W - the arousal degree of the driver;

T - the driver's arousal duration;

Q - visual stimulus quantity in the fatigue arousal section.

$$Q=I*t \qquad\qquad （9）$$

where Q -- visual stimulus quantity of tunnel fatigue arousal section (cd · s/m$^2$) ;

I - visual stimulus intensity of tunnel fatigue arousal section, namely the color and luminance of fatigue arousal section (cd/m$^2$) ;

t - the stimulus duration of the tunnel, i.e., the driving time (s) of the driver in the tunnel fatigue arousal section.

Here, the following experimental implementation example is proposed, which can be used for determining the length and spacing of fatigue arousal sections. The experiment may be used to obtain the aforementioned third experimental data for generating the fourth and fifth samples.

1. Experimental Setup

In the experiment presented in this example, to study the stimulus quantity and stimulus duration for driver fatigue arousal, the physiological and psychological indicators of 12 drivers before and after the arousal of 45 stimulus levels, including 3 colors, 3 luminance and 5 different stimulus duration, by simulating the environmental conditions in the middle portion of the tunnel. The study aims to establish the correlation between the amount of arousal and the degree of arousal and the duration, and to analyze the demand of driver fatigue arousal stimulus. Based on the stimulus quantity demand, the arousal degree and the stimulus duration demand corresponding to the arousal duration are determined under fixed color and luminance conditions. The set length of the fatigue arousal section in the tunnel with different design speeds is proposed.

2. Selection of Experimental Parameters

To study and propose the length setting of fatigue arousal sections, three monochromatic colors (blue, purple, and cyan) were selected, with side wall luminance of 1.5 cd/m $^2$,1.7cd/m$^2$,1.8cd/m$^2$ , and stimulus durations of 4s, 6s, 8s, 10s and 12s, respectively, , totaling 45 stimulus quantity level conditions. The physiological and psychological indicators before and after the fatigue arousal section are collected from 12 drivers to generate the arousal degree and duration of the drivers.

3. Experimental procedure

To ensure synchronization of each experiment, the time of each instrument may be accurately calibrated before each experiment. Before the experiment begins, drivers are fully familiarized with the experimental process and requirements. The specific implementation steps of the experiment are as follows:

(1) The lighting fixtures in the simulated tunnel are adjusted to achieve a road surface luminance of 1 cd/m$^2$, and a luminance of the side wall of 0.5 cd/m$^2$, ensuring uniformity of road surface luminance.

(2) The test driver visually recognizes the lighting environment in the simulated middle portion of the tunnel for 3 minutes with the wall washer lights turned off, simulating the driving state in the tunnel middle section environment.

(3) After simulating in the middle section environment, the wall washer lights are turned on and adjusted to blue fatigue arousal section, with the fatigue arousal section side wall luminance set to 1.5 cd/m$^2$.

(3) The color luminance meter is used to measure the tunnel side wall luminance and wavelength, with the current color and luminance stimulus arousal of the wall washer lights maintained for 4s.

(4) The wall washer lights are turned off, and the driver resumes visual recognition in the simulated middle portion tunnel lighting environment for 3 minutes. Then the wall washer lights are turned on, keeping the color and luminance unchanged, with the wall washer light stimulus arousal maintained for 6s.

(5) The step (5) is repeated until the completion of the arousal experiment of the colors and luminance for the current fatigue arousal section.

(6) It changes to the next test driver and the steps (2) ~ (6) are repeated until all 12 test drivers complete the arousal experiment for all stimulus durations at a color of blue and side wall luminance of 1.5 cd/m$^2$.

(7) It changes to the next set of wall washing lights, the steps (2) ~ (6) are repeated until 12 drivers complete the arousal experiment for three colors of fatigue arousal sections and a side wall luminance of 1.5 cd/m$^2$ for all stimulus durations.

(8) The steps (1) ~ (7) are repeated to conduct the fatigue arousal section of side wall luminance experiments until 12 test drivers complete the arousal experiment for all stimulus levels.

In this experiment, in addition to the luminance for the fatigue arousal section, two other luminance are also tested. However, it may be conceivable that in an embodiment of this application, only one luminance of the fatigue arousal section and the luminance of the multiple different values for the fatigue arousal section, may be tested. If one luminance of the fatigue arousal section is tested, the above step (8) can be omitted.

4. Analysis of Length Setting Requirements for Fatigue Arousal Sections

In some embodiments, physiological and psychological indicator data, such as heart rate variability (HRV) data in form of LF (low frequency)/HF (high frequency) data are obtained.

In the experiment, the physiological and psychological indicator data of 12 test drivers under 45 different stimulus conditions, including 3 colors, 3 luminance and 5 stimulus durations, are obtained. The physiological and psychological parameters under different stimulus combinations were processed to obtain the LF/HF data for each driver in each experiment period. After excluding invalid and abnormal data according to the driver gaze point information recorded by the eye tracker, a total of 102475 data samples were obtained.

1) Correlation between Arousal Degree and Stimulus Quantity

In some embodiments, the product of the luminance and the stimulus duration gives the stimulus quantity, the correlation between the stimulus quantity and the degree of driver fatigue. As an explanation rather than a limitation, the driver's arousal degree shows a gradual increasing trend with the increase of fatigue arousal section stimulus quantity, indicating an obvious positive correlation between arousal degree and fatigue arousal section stimulus quantity. As the fatigue arousal section stimulus quantity increases, the increasing rate of arousal degree slows down, and due to drivers' physiological and psychological thresholds, the growth rate of arousal degree will continue to slow down until it no longer increases with increasing stimulus quantity.

In some embodiments, Pearson correlation analysis yielded a partial correlation coefficient of 0.959 between stimulus quantity and arousal degree. Under the three colors (blue, purple, and cyan) of fatigue arousal section environments, the stimulus quantity of the fatigue arousal section and the arousal degree have a logarithmic relationship. By fitting the data of fatigue arousal section stimulus quantity and arousal degree, a correlation model between fatigue arousal section stimulus quantity and arousal degree under the three color conditions was established as shown in equation (10), with the fitting curve of stimulus quantity and arousal degree shown in Figure 19.

$$W = 0.7848\ \ln(Q) + 0.3898 \qquad (10)$$

where W - arousal degree;
Q -- stimulus quantity of fatigue arousal section in blue, purple, cyan colors

The driver's arousal degree shows a gradual increasing trend with the increase of fatigue arousal section stimulus quantity, but after the stimulus quantity increases to a certain extent, the growth rate of arousal degree slows down; and due to the stimulation of different colors in the fatigue arousal section, the driver's arousal degree after stimulation is definitely greater than 0. In an embodiment, the given driver arousal degree LF/HF change rate index value is required to reach 2.1, i.e., W=2.1, to ensure that the fatigue arousal section stimulus quantity can arouse drivers to an alert state. Substituting W=2.1 into equation (10), the minimum stimulus quantity needed to arouse drivers to an alert state is obtained as Q=8.84cd·s/m$^2$. In the specific embodiment, the above arousal degree threshold of W=2.1 may be determined based on the arousal degree threshold used for determining colors in the previous second experiment. However, it may be envisioned that when determining the minimum stimulus quantity based on the arousal degree model, other arousal degree thresholds may be applicable, which falls within the scope of the invention.

In the embodiment, the above fitting curve does not distinguish among colors.

2) Correlation between Fatigue Arousal Section Stimulus Quantity and Arousal Duration

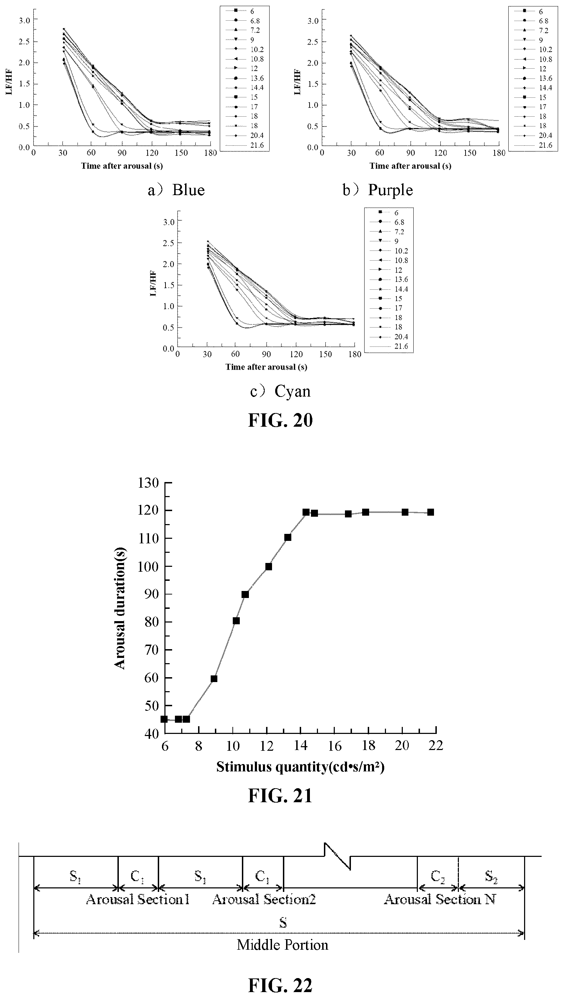

In the embodiment of the disclosure, various manners or indicators may be used to determine the arousal duration endpoint. In some embodiments, the arousal duration after different stimulus quantities may be calculated based on the time it took for drivers to experience fatigue again. In a specific embodiment, the HRV data of the driver, such as the change pattern of HF/LF indicators, may be used. In a more specific embodiment, all drivers' LF/HF after arousal may be obtained in real time or at given time intervals, such as every 30s, to obtain the change patterns of LF/HF data after arousal by the fatigue arousal sections of three colors with different stimulus quantities when drivers return to the ordinary middle section environment of the simulated tunnel, as shown in Figure 20. In a particularly preferred embodiment, the arousal duration endpoint may be determined based on the change pattern. More preferably, based on the change pattern shown in Figure 20, the steady-state (plateau) data in the change pattern diagram may be used to roughly determine the HRV threshold, such as the LF/HF index threshold, used for determining the endpoint of arousal duration (arousal (fatigue) critical point). As a result, when the LF/HF data of drivers after arousal from different stimulus quantities of the fatigue arousal section reached the previously determined HRV threshold value, it may be confirmed as the endpoint of arousal duration.

In one example, the correlation between arousal duration and fatigue arousal section stimulus quantity determined from data obtained when drivers were in a stationary state is shown in Figure 21. As shown in Figure 21, drivers may achieve an arousal duration of more than 45s when they receive arousal stimuli exceeding a certain stimulus quantity. Within a certain stimulus quantity range, drivers' arousal duration remains constant, and it begins to increase after exceeding a certain stimulus quantity. According to stimulus theory, this stimulus quantity is the minimum stimulus quantity. As the stimulus quantity increases, drivers' arousal duration increases, but when the stimulus quantity reaches $14.4cd \cdot s/m^2$, the arousal duration reaches a maximum value of 120s and does not continue to increase with further increases in stimulus quantity. The time for driving fatigue to occur in drivers in a static tunnel middle portion environment may be determined to be approximately 120s.

As an explanation rather than a limitation, considering that the correlation between arousal duration and stimulus quantity conforms to the distribution pattern of the logistic function model in discrete choice method. By performing multivariate logistic regression analysis on the fatigue arousal section stimulus quantity and arousal duration data, thus, according to the different times when drivers experience fatigue in the middle section of the tunnel at different operating speeds, the correlation models (arousal duration models) between tunnel fatigue arousal section stimulus quantity and arousal duration under dynamic conditions are shown in equations (11), (12), and (13) respectively.

① Speed of 60km/h

$$T_1 = \begin{cases} 54.97502 & 8.84 \leq Q_1 < 10.43 \\ 147.976 - \dfrac{95.24837}{1+(\dfrac{Q_1}{12.2655})^{8.67823}} & 10.43 \leq Q_1 < 16.99 \\ 146.6 & Q_1 \geq 16.99 \end{cases} \qquad (11)$$

where $T_1$-- driver's arousal duration after the fatigue arousal section at speed 60km/h;
$Q_1$- stimulus quantity of fatigue arousal section at speed 60km/h.

② Speed of 80km/h

$$T_2 = \begin{cases} 64.13252 & 9.42 \leq Q_2 < 13.877 \\ 172.6251 - \dfrac{111.1144}{1+(\dfrac{Q_2}{16.31935})^{8.67823}} & 13.877 \leq Q_2 < 22.608 \\ 171.02 & Q_2 \geq 22.608 \end{cases} \qquad (12)$$

where $T_2$-- driver's arousal duration after the fatigue arousal section at speed 80km/h;
$Q_2$- stimulus quantity of fatigue arousal section at speed 80km/h.

③ Speed of 100km/h

$$T_3 = \begin{cases} 76.95752 & 17.64 \leq Q_3 < 25.987 \\ 207.1461 - \dfrac{133.3347}{1+(\dfrac{Q_2}{30.5598})^{8.67823}} & 25.987 \leq Q_3 < 42.336 \\ 205.22 & Q_3 \geq 42.336 \end{cases} \qquad (13)$$

where $T_3$-- driver's arousal duration after the fatigue arousal section at speed 100km/h;
$Q_3$- stimulus quantity of fatigue arousal section at speed 100km/h.

In the embodiments of the disclosure, the minimum stimulus quantity determined by the aforementioned arousal degree and stimulus quantity correlation model (arousal level model) may be used to constrain the arousal duration model. For example, in this example, the lower endpoint value of the stimulus quantity range in the first plateau segment of the arousal duration model at a speed of 60km/h is constrained by the minimum stimulus quantity.

3) Length Setting of Fatigue Arousal Sections

As an explanation rather than a limitation, the length setting, number, and spacing of fatigue arousal sections in long tunnel depend on the length of the middle section. In the embodiments of the disclosure, when arranging fatigue arousal section(s) in the long tunnel, the stimulus quantity may fulfill drivers' maximum arousal duration as much as possible to minimize the number of fatigue arousal sections. The maximum arousal duration is the time when drivers first experience driving fatigue after entering the middle portion of a long tunnel at the different speeds. In a preferred embodiment of the disclosure, the end-portion fatigue arousal section is also set in this way, with its stimulus quantity depending on the length of the remaining middle portion after drivers experience driving fatigue again after being stimulated by previous middle-portion fatigue arousal section(s). The arousal duration corresponding to the stimulus quantity should ensure that drivers can complete driving through the remaining middle section.

As shown in Figure 22, a schematic diagram of fatigue arousal section arrangement in long tunnels is illustrated. It lets S1 be the spacing (first spacing) of non-end-portion fatigue arousal sections, which is the driving distance at which drivers experience fatigue at different operating speeds; C1 be the set length (first length) of non-end-section fatigue arousal sections; S2 be the remaining tunnel length after drivers experience driving fatigue again, which serves as the spacing between the end-section middle section and the tunnel exit (second spacing); C2 be the set length (second length) of the end-section fatigue arousal section.

(1) Length Setting of Several First Fatigue Arousal Sections in Long Tunnel

In this example, according to the time when the driver to experience fatigue at running speed of 60 km/h, 80 km/h and 100km/h respectively, the stimulus duration when the driver meets the arousal time of 146.6s, 171.02s and 205.22s respectively. By substituting $T_1$=146.6s, $T_2$=171.02s, $T_3$=205.22s into equations (11), (12) and (13) respectively, when the speed is 60 km/h, 80 km/h and 100km/h respectively, the required stimulus quantity of driving fatigue arousal section using the three colors of blue, purple and green in the long tunnel should be 16.992cd · s/m$^2$,22.608cd·s/m$^2$,42.336cd·s/m$^2$ respectively.

According to Table 3, the maximum luminance that can be set in the side wall of the fatigue arousal section in the long tunnel at speeds of 60 km/h, 80 km/h and 100 km/h should be 1.8 cd/m$^2$2.7cd/m$^2$,5.4cd/m$^2$, respectively. Substituting T1=146.6s, T2=171.02s, T3=205.22s into equations (11), (12), (13) respectively, it may obtain that when using blue, purple, and cyan colors, the required stimulus quantities for fatigue arousal sections at speeds of 60km/h, 80km/h, and 100km/h should be 16.992cd·s/m$^2$, 22.608cd·s/m$^2$, and 42.336cd·s/m$^2$ respectively. According to Table 3, the maximum settable side wall luminance for fatigue arousal sections in the long tunnel at speeds of 60km/h, 80km/h, and 100km/h should be 1.8cd/m$^2$, 2.7cd/m$^2$, and 5.4cd/m$^2$ respectively. Substituting Q=16.992cd·s/m$^2$, 22.608cd·s/m$^2$, 42.336cd·s/m$^2$ and I=1.8cd/m$^2$, 2.7cd/m$^2$, 5.4cd/m$^2$ into equation (9), it may obtain the required stimulus duration for drivers traveling at different operating speeds in long tunnel fatigue arousal sections using blue, purple, and cyan as main colors. Finally, calculating the driving distance corresponding to different operating speeds' stimulus duration gives the required fatigue arousal section length C1, as shown in Table 5.

Table 5: **Length Setting of First Several Fatigue Arousal Sections in Long Tunnel**

| Design speed (km/h) | Luminance of side wall in the fatigue arousal section(cd/m$^2$) | Stimulus duration (s) | Set length of the fatigue arousal sections(m) |
|---|---|---|---|
| **60** | 1.8 | 9.44 | 157.333 |
| **80** | 2.7 | 8.73 | 186.074 |
| **100** | 5.4 | 7.84 | 217.778 |

In this example, in the long tunnel with the operating speed of 60 km/h, the fatigue arousal section set with the primary colors of blue, purple and cyan, with the side wall luminance of the fatigue arousal section of 1.8 cd/m$^2$, the length of the fatigue arousal section is set to 160m. In the long tunnel with the operating speed of 80km/h, the fatigue arousal section set with the primary colors of blue, purple and cyan, with the side wall luminance of the fatigue arousal section of 2.7 cd/m$^2$, the length of the fatigue arousal section is 190m. In the long tunnel with the operating speed of 100km/h, the fatigue arousal section set with the primary colors of blue, purple and cyan, with the side wall luminance of the fatigue arousal section of 5.4

cd/m$^2$, the length of the fatigue arousal section is set to 220m. For long tunnels requiring more than one fatigue arousal section, the first several fatigue arousal sections can be arranged with spacing S1 according to the driving distance at which drivers experience driving fatigue.

(2) Length Setting of the Last Fatigue Arousal Section in Long Tunnel

In the example, when setting the last fatigue arousal section in the middle section of long tunnels, calculations need to be performed according to equations (11), (12), and (13) based on the length of the remaining middle portion. When the operating speeds are 60km/h, 80km/h, and 100km/h respectively, the minimum arousal duration corresponds to calculated stimulus durations that need to reach 3.93s, 3.49s, and 3.27s respectively, meaning that when the fatigue arousal section lengths reach 65.56m, 77.53m, and 90.74m, drivers can drive distances of 916.25m, 1425.167m, and 2137.708m before experiencing driving fatigue again.

When the drivers are driving from the middle portion of the tunnel to the tunnel exit portion, they usually experience a dark-light transition change in the light environment, and the driver's driving workload increases, which results in a certain degree of arousal to the driver. Therefore, when setting the last fatigue arousal section, if the remaining middle portion length S2 is less than the driving distance S1 of the driver at the different design speeds, but greater than the driving distance at which drivers experience fatigue after the minimum stimulus as mentioned above, then the required stimulus quantity and stimulus duration need to be calculated based on the length S2 of the remaining tunnel mediate section according to Equations (11), (12), and (13) to determine the set length C2 of the fatigue arousal section.

If the remaining middle portion length S2 is not greater than the above-mentioned driving distance at which drivers would experience driving fatigue after being aroused by the minimum stimulus quantity of the fatigue arousal section, then the fatigue arousal section needs to be set according to the minimum stimulus quantity calculated above. That is, assuming that blue, purple, and cyan are selected as main colors for the fatigue arousal section, at the operating speeds of 60km/h, 80km/h, and 100km/h, with corresponding maximum luminance of 1.8cd/m$^2$, 2.7cd/m$^2$, and 5.4cd/m$^2$, the fatigue arousal section setting lengths will be 65m, 80m, and 90m respectively.

In the example, the starting arrangement position of fatigue arousal section mentioned in other embodiment is not shown. However, it may be envisioned that in some embodiments, the setting of fatigue arousal section length and spacing can be done without combining with the starting arrangement position, such as having the first fatigue arousal section immediately follow the entrance section or transition section, or at a given distance from the tunnel entrance, such as setting the first fatigue arousal section at the first spacing. In some other preferred embodiments, the setting of fatigue arousal section length and spacing may be combined with this starting arrangement position, thus having the first fatigue arousal section begin at the starting arrangement position.

In the embodiment of the disclosure provided is a method for arranging and/or calculating technical parameters of a fatigue arousal section in a road tunnel, particularly an expressway long tunnel, which may be used to obtain fatigue arousal section design and/or technical parameters that meet drivers' physiological and psychological needs. On one hand, in the embodiments of this invention it may start from drivers' physiological and psychological needs to obtain effective arrangements and/or technical parameters for road tunnel, particularly expressway long tunnel fatigue arousal sections through scientific, effective, and refined models. On the other hand, while being widely applicable to various types of road tunnels, it may still specifically assist in creating good, reliable, and safe visual environments for fatigue arousal sections, solving the driving safety risk issues caused by driver fatigue in the middle portions of various tunnels, particularly in the long tunnels.

Unless explicitly indicated, the actions or steps of methods and programs recorded according to the embodiments of this invention do not necessarily need to be executed in a specific order to achieve the desired results. In some embodiments, multitasking and parallel processing are also possible or may be advantageous.

In this document, multiple embodiments of the disclosure are described, but for simplicity, the description of the embodiments may not be exhaustive, and the same or similar features or parts among different embodiments may be omitted. In this document, "an embodiment," "some embodiments," "examples," "specific examples," or "some examples," apply to at least one embodiment or example according to the disclosure, not necessarily all embodiments. These terms do not necessarily mean reference to the same embodiment or example. Without being contradictory, those skilled in the art may combine and assemble different embodiments or examples and their features.

The exemplary system and method of the disclosure have been specifically illustrated and described with reference to the above embodiments, which are only examples of the best mode for implementing the present system and method. Those skilled in the art may understand that various changes to the embodiments of the system and methods described herein can be made in implementing the system and/or method without departing from the spirit and scope of the disclosure defined in the attached claims.

**Claims**

1.  A method for arranging a fatigue arousal section in a tunnel, comprising:

    S130: determining a plurality of colors and luminance of fatigue arousal section;
    S160: based on a plurality of fifth samples, generating a plurality of arousal duration models in which the drivers' arousal duration changes with stimulus quantity at multiple different vehicle speeds, wherein the fifth samples comprise arousal durations of different drivers under different stimulus quantities at the plurality of colors and luminance;
    S170: determining length and spacing of fatigue arousal section to be arranged according to a specified speed of the tunnel, using the arousal duration models; and
    S180: arranging one or more fatigue arousal sections in the tunnel based on the determined length and spacing of the fatigue arousal section.

2.  The method for arranging the fatigue arousal section in the tunnel according to claim 1, wherein each of the arousal duration models has a first plateau segment where the arousal duration remains roughly constant with changing stimulus quantity, a second rising segment where the arousal duration increases with increasing stimulus quantity, and a third plateau segment where the arousal duration remains roughly constant with changing stimulus quantity.

3.  The method for arranging the fatigue arousal section in the tunnel according to claim 2, wherein each arousal duration model is as follows:

$$T_i = \begin{cases} t0 & q0 \le Q_i < q1 \\ t1 - \dfrac{a}{1 + (\dfrac{Q_1}{b})^c} & q1 \le Q_i < q2 \\ t2 & Q_i \ge q2 \end{cases}$$

    where T is the arousal duration, Q is the stimulus quantity, t0, t1, t2, a, b, c are the model parameters, q0, q1, q2 are the endpoint value of the stimulus quantity range, i refers to the serial number of the arousal duration model, the model parameters and endpoint values are different according to the arousal duration model.

4.  The method for arranging the fatigue arousal section in the tunnel according to claim 3, wherein the plurality of arousal duration models are respectively represented as follows:

    ① for a first speed:

$$T_1 = \begin{cases} 54.97502 & 8.84 \le Q_1 < 10.43 \\ 147.976 - \dfrac{95.24837}{1 + (\dfrac{Q_1}{12.2655})^{8.67823}} & 10.43 \le Q_1 < 16.99 \\ 146.6 & Q_1 \ge 16.99 \end{cases}$$

    where $T_1$- driver's arousal duration after the fatigue arousal section at the first speed;
    $Q_1$-- the stimulus quantity of fatigue arousal section at the first speed
    ② for a second speed:

$$T_2 = \begin{cases} 64.13252 & 9.42 \le Q_2 < 13.877 \\ 172.6251 - \dfrac{111.1144}{1 + (\dfrac{Q_2}{16.31935})^{8.67823}} & 13.877 \le Q_2 < 22.608 \\ 171.02 & Q_2 \ge 22.608 \end{cases}$$

where $T_2$-- driver's arousal duration after the fatigue arousal section at the second speed;
$Q_2$-- the stimulus quantity of fatigue arousal section at the second speed; and
③ for a third speed:

$$T_3 = \begin{cases} 76.95752 & 17.64 \le Q_3 < 25.987 \\ 207.1461 - \dfrac{133.3347}{1 + (\dfrac{Q_2}{30.5598})^{8.67823}} & 25.987 \le Q_3 < 42.336 \\ 205.22 & Q_3 \ge 42.336 \end{cases}$$

where $T_3$-- driver's arousal duration after the fatigue arousal section at the third speed;
$Q_3$-- the stimulus quantity of fatigue arousal section at the third speed.

5. The method for arranging the fatigue arousal section in the tunnel according to claim 2, wherein the step S170 comprises:

S171: determining the arousal duration model corresponding to the specified speed;
S172: obtaining the arousal duration and minimum stimulus quantity corresponding to the third plateau segment of the determined arousal duration model;
S173: determining a first length of the fatigue arousal section based on the minimum stimulus quantity corresponding to the third plateau segment, the color, and the luminance; and
S174: determining a first spacing of the fatigue arousal section based on the arousal duration.

6. The method for arranging the fatigue arousal section in the tunnel according to claim 5, wherein the step S170 further comprises:

S175: obtaining the length of the tunnel; and
S176: based on the tunnel length, the first length, and the first spacing, determining a second length and a second spacing for the end-portion fatigue arousal section using the determined arousal duration model;
the step S180 comprises: arranging middle-portion fatigue arousal sections in the tunnel's middle portion based on the first length and the first spacing, and arranging an end-portion fatigue arousal section in the tunnel's end portion based on the second length and second spacing.

7. The method for arranging the fatigue arousal section in the tunnel according to claim 1, wherein the step S160 comprises:

S161: based on the plurality of fifth samples obtained at a reference vehicle speed, generating a reference arousal duration model depicting how the driver's arousal duration changes with stimulus quantity at the reference vehicle speed, wherein the reference speed may be zero, i.e., in a stationary state; and
S162: processing the reference arousal duration model using regression analysis algorithms to generate the plurality of arousal duration models corresponding to the multiple different vehicle speeds.

8. The method for arranging the fatigue arousal section in the tunnel according to claim 1, further comprising:

S150: based on a plurality of fourth samples, generating an arousal level model in which the driver's arousal level changes with stimulus quantity, wherein the fourth samples comprise the arousal levels of different drivers at different stimulus quantities with the plurality of colors and luminance;

the step S160 comprises: constraining the plurality of arousal duration models using a minimum stimulus quantity determined by the arousal level model.

9. The method for arranging the fatigue arousal section in the tunnel according to claim 8, further comprising:
S140: obtaining third experimental data for generating the fourth and fifth samples, comprising:

S141: instructing one of multiple experimental drivers to visually recognize in the lighting environment in a middle portion of the tunnel for a fourth predetermined duration to collect fourth heart rate variability data in a non-stimulated state;
S142: stimulating the experimental driver to induce arousal by simulating a fatigue arousal section with one of the plurality of colors and the luminance for one of multiple fifth predetermined durations, and collecting fifth heart rate variability data in the stimulated state;
S143: deactivating the simulated fatigue arousal section to allow the experimental driver to resume visual recognition in the lighting environment in the middle portion of the tunnel for a sixth predetermined duration, and collecting sixth heart rate variability data in the aroused state;
S144: repeating steps S142 and S143 for each fifth predetermined duration;
S145: repeating steps S141 to S144 for each experimental driver; and
S146: repeating steps S141 to S145 for each color,
wherein the fourth samples are obtained from the fourth and fifth heart rate variability data; the fifth samples are obtained at least from the sixth heart rate variability data.

10. The method for arranging the fatigue arousal section in the tunnel according to claim 1, wherein the step S130 comprises:

S131: obtaining second experimental data for generating third samples;
S132: processing the second experimental data using an arousal level quantification model to obtain the third samples, wherein the third samples comprise the arousal levels of different drivers under different colors;
S133: determining an arousal level threshold based on the third samples; and
S134: selecting the plurality of colors based on the determined luminance and the arousal level threshold.

11. The method for arranging the fatigue arousal section in the tunnel according to claim 10, wherein the step S131 comprises:

S1311: instructing one of multiple experimental drivers to visually recognize the lighting environment in the middle portion of the tunnel for a first predetermined duration to collect first heart rate variability data in a non-stimulated state;
S1312: stimulating the experimental driver to induce arousal by simulating a fatigue arousal section using one of the colors from a color library and one of the luminance from a luminance group for a second predetermined duration, and collecting second heart rate variability data in the stimulated state;
S1313: deactivating the simulated fatigue arousal section to allow the experimental driver to resume visual recognition in the lighting environment in the middle portion of the tunnel for a third predetermined duration;
S1314: repeating steps S1311 to S1313 for each color in the color library;
S1315: repeating steps S1311 to S1314 for each experimental driver;
S1316: repeating steps S1311 to S1315 for each luminance in the luminance group,
wherein the third samples are obtained based on the first and second heart rate variability data, and the number of colors in the color library is greater than or equal to the number of the selected plurality of colors.

12. The method for arranging the fatigue arousal section in the tunnel according to claim 1, further comprising:

S110: determining a starting arrangement position of the fatigue arousal section according to a determined driving fatigue threshold and the specified speed;
wherein the step S180 comprises: arranging one or more fatigue arousal sections in the tunnel based on the starting arrangement position, the length and the spacing of the fatigue arousal section.

13. The method for arranging the fatigue arousal section in the tunnel according to claim 12, wherein the step S110 comprises:

S111: determining length of at least one entrance portion;

S112: determining length of at least one transition portion;

S113: determining a driving distance when the driver experiences fatigue in the middle portion of the tunnel based on the driving fatigue threshold and the specified speed; and

S114: determining the starting arrangement position based on the length of the at least one entrance portion, the length of the at least one transition portion, and the driving distance.

14. The method for arranging the fatigue arousal section in the tunnel according to claim 13, wherein the step S113 comprises:

S1132: determining the driving fatigue threshold based on a plurality of first samples, according to a given model of driving fatigue level versus heart rate variability, wherein the first samples comprise heart rate variability of different drivers driving in a calm state and when feeling fatigued in the tunnel environment;

S1133: generating a plurality of fatigue level models depicting changes in drivers' driving fatigue level over time at different vehicle speeds, based on a plurality of second samples, wherein the second samples comprise driving fatigue levels of different drivers at different times when driving in the tunnel environment at different vehicle speeds; and

S1134: determining the driving distance when the driver experiences fatigue in the middle portion of the tunnel using the fatigue level models, based on the driving fatigue threshold and the specified speed.

15. The method for arranging the fatigue arousal section in the tunnel according to claim 14, wherein the model of driving fatigue level versus heart rate variability is represented as follows:

$$A_{ij} = \left[ \left( \frac{LF}{HF} \right)_{ij} - \left( \frac{LF}{HF} \right)_i \right] \Big/ \left( \frac{LF}{HF} \right)_i$$

where $A_{ij}$ -- driving fatigue of i$^{th}$ driver at driving time j in the tunnel;

$\left( \dfrac{LF}{HF} \right)_{ij}$ -- the HRV value of i$^{th}$ driver at the j$^{th}$ moment while driving in the tunnel;

$\left( \dfrac{LF}{HF} \right)_i$ -- the HRV value of i$^{th}$ driver in calm state;

the multiple fatigue level models are represented as follows:

① for a first speed

$$A = 1.80869 + \frac{163.00287}{104.6879 \sqrt{\dfrac{\pi}{2}}} * e^{-2\left( \frac{t - 146.7103}{104.6879} \right)^2}$$

② for a second speed

$$A = 1.91254 + \frac{181.81534}{124.46489 \sqrt{\dfrac{\pi}{2}}} * e^{-2\left( \frac{t - 174.20238}{124.46489} \right)^2}$$

③ for a third speed

$$A = 2.01221 + \frac{192.91589}{146.19899\sqrt{\dfrac{\pi}{2}}} * e^{-2\left(\frac{t-226.83989}{146.19899}\right)^2}$$

where A -- the driving fatigue level of drivers in the tunnel environment;
t -- the driving time of the driver in the tunnel environment.

16. The method for arranging the fatigue arousal section in the tunnel according to claim 14, wherein the step S113 further comprises:
S1131: obtaining first experimental data for generating the first and second samples, specifically comprising:

S11311: collecting heart rate variability data of one of multiple experimental drivers in a calm state;
S11312: instructing the experimental driver to drive the vehicle into the tunnel at one of a plurality of experimental speeds, and collecting the driver's heart rate variability data in real time;
S11313: recording the moment when the experimental driver feels fatigued during the driving process, for determining the heart rate variability data of the driver at that moment; and
S11314: repeating steps S11311 to S11313 for each experimental driver and each experimental speed.

17. The method for arranging the fatigue arousal section in the tunnel according to claim 1, further comprising:

S120: determining an arrangement height of the fatigue arousal section based on the determined driver's eye height and the specified speed;
wherein the step S180 comprises: arranging the one or more fatigue arousal sections in the tunnel based on the arrangement height, the length, and the spacing of the fatigue arousal section.

18. The method for arranging the fatigue arousal section in the tunnel according to claim 1, wherein the fatigue arousal section comprises wall washer lights alternating in the plurality of colors installed on both sides of the road, with the wall washer lights having the specified luminance.

19. A device for arranging a fatigue arousal sections in a tunnel, comprising:

a first determination unit, configured to determine a plurality of colors and luminance for the fatigue arousal section;
a generation unit, configured to generate a plurality of arousal duration models based on a plurality of fifth samples in which the drivers' arousal duration changes with stimulus quantity at multiple different vehicle speeds, the fifth samples comprising arousal durations of different drivers under different stimulus quantities at the plurality of colors and luminance;
a second determination unit, configured to determine length and spacing of the fatigue arousal section to be arranged according to the specified speed of the tunnel using the arousal duration models; and
an arrangement unit, configured to arrange one or more fatigue arousal sections in the tunnel based on the determined length and spacing of the fatigue arousal section.

20. A road tunnel, comprising a fatigue arousal section arranged according to a method for arranging a fatigue arousal section in a tunnel, the method for arranging the fatigue arousal section in the tunnel comprising:

determining a plurality of colors and luminance of fatigue arousal section;
based on a plurality of samples, generating a plurality of arousal duration models in which the drivers' arousal duration changes with stimulus quantity at multiple different vehicle speeds, wherein the samples comprise arousal durations of different drivers under different stimulus quantities at the plurality of colors and luminance;
determining length and spacing of fatigue arousal section to be arranged according to a specified speed of the tunnel, using the arousal duration models; and
arranging one or more fatigue arousal sections in the tunnel based on the determined length and spacing of the fatigue arousal section.

S130

Determine a plurality of colors and luminance of fatigue arousal section

S160

Generate a plurality of arousal duration models based on a plurality of fifth samples, in which the drivers' arousal duration changes with stimulus quantity at multiple different vehicle speeds

S170

Determine length and spacing of fatigue arousal section to be arranged according to a specified speed of the tunnel, using the arousal duration models

S180

Arrange one or more fatigue arousal sections in the tunnel based on the determined length and spacing of the fatigue arousal section

**FIG. 1**

Determine the arousal duration model corresponding to the specified vehicle speed — S171

Obtain the arousal duration and minimum stimulus quantity corresponding to the third plateau segment of the determined arousal duration model — S172

Determine a first length of the fatigue arousal section based on the minimum stimulus quantity corresponding to the third plateau segment, the color, and the luminance — S173

Determine a first spacing of the fatigue arousal section based on the arousal duration — S174

Obtain the length of the tunnel — S175

Determine a second length and a second spacing for the end-section fatigue arousal section based on the tunnel length, the first length, and the first spacing, using the determined arousal duration model — S176

**FIG. 2**

Based on the plurality of fifth samples obtained at a reference vehicle speed, generate a reference arousal duration model depicting how the driver's arousal duration changes with stimulus quantity at the reference vehicle speed — S161

Process the reference arousal duration model using regression analysis algorithms to generate the plurality of arousal duration models corresponding to the multiple different vehicle speeds — S162

**FIG. 3**

Determine a starting arrangement position of the fatigue arousal section according to a determined driving fatigue threshold and a specified speed

S110

Determine an arrangement height of the fatigue arousal section based on the determined driver's eye height and the specified speed

S120

Determine a plurality of colors and luminance of fatigue arousal section based on a determined arousal level threshold

S130

Obtain third experimental data for generating the fourth and fifth samples

S140

Generate an arousal level model based on a plurality of fourth samples, in which the driver's arousal level changes with stimulus quantity

S150

Generate a plurality of arousal duration models based on a plurality of fifth samples, in which the drivers' arousal duration changes with stimulus quantity at multiple different vehicle speeds

S160

Determine length and spacing of fatigue arousal section to be arranged according to the specified speed of the tunnel, using the arousal duration models

S170

Arrange one or more fatigue arousal sections in the tunnel based on the determined length and spacing of the fatigue arousal section

S180

**FIG. 4**

Instruct one of multiple experimental drivers to visually recognize the lighting environment in a middle portion of the tunnel for a fourth predetermined duration to collect fourth heart rate variability data in a non-stimulated state — S141

Stimulate the experimental driver to induce arousal by simulating a fatigue arousal section with one of the plurality of colors and the luminance for one of multiple fifth predetermined durations, and collect fifth heart rate variability data in the stimulated state — S142

Deactivate the simulated fatigue arousal section to allow the experimental driver to resume visual recognition in the lighting environment in the middle portion of the tunnel for a sixth predetermined duration, and collect sixth heart rate variability data in the aroused state — S143

Repeat steps S142 and S143 for each fifth predetermined duration — S144

Repeat steps S141 to S144 for each experimental driver — S145

Repeat steps S141 to S145 for each color — S146

**FIG. 5**

Obtain second experimental data for generating third samples — S131

Process the second experimental data using an arousal level quantification model to obtain the third samples — S132

Determine an arousal level threshold based on the third samples — S133

Select a plurality of colors based on the determined luminance and the arousal level threshold — S134

**FIG. 6**

Instruct one of multiple experimental drivers to visually recognize the lighting environment in the middle portion of the tunnel for a first predetermined duration to collect first heart rate variability data in a non-stimulated state — S1311

Stimulate the experimental driver to induce arousal by simulating a fatigue arousal section using one of the colors from a color library and one of the luminances from a luminance group for a second predetermined duration, and collect second heart rate variability data in the stimulated state — S1312

Deactivate the simulated fatigue arousal section to allow the experimental driver to resume visual recognition in the lighting environment in the middle portion of the tunnel for a third predetermined duration — S1313

Repeat steps S1311 to S1313 for each color in the color library — S1314

Repeat steps S1311 to S1314 for each experimental driver — S1315

Repeat steps S1311 to S1315 for each luminance in the luminance group — S1316

**FIG. 7**

```
┌─────────────────────────────────────────────────┐  S111
│                                                 │ ⌇
│     Determine a length of at least one entrance portion
│                                                 │
└─────────────────────────────────────────────────┘
                        │
                        ▼
┌─────────────────────────────────────────────────┐  S112
│                                                 │ ⌇
│     Determine a length of at least one transition portion
│                                                 │
└─────────────────────────────────────────────────┘
                        │
                        ▼
┌─────────────────────────────────────────────────┐  S113
│  Determine a driving distance when the driver experiences │ ⌇
│  fatigue in the middle portion of the tunnel based on the │
│     driving fatigue threshold and the specified speed     │
└─────────────────────────────────────────────────┘
                        │
                        ▼
┌─────────────────────────────────────────────────┐  S114
│  Determine the starting arrangement position based on the │ ⌇
│  length of the at least one entrance portion, the length of the at │
│   least one transition portion, and the driving distance  │
└─────────────────────────────────────────────────┘
```

**FIG. 8**

```
┌─────────────────────────────────────────────────┐  S1131
│  Obtain first experimental data for generating the first and │ ⌇
│                   second samples                  │
└─────────────────────────────────────────────────┘
                        │
                        ▼
┌─────────────────────────────────────────────────┐  S1132
│  Determine the driving fatigue threshold based on a plurality │ ⌇
│  of first samples, according to a given model of driving fatigue │
│        level versus heart rate variability        │
└─────────────────────────────────────────────────┘
                        │
                        ▼
┌─────────────────────────────────────────────────┐  S1133
│  Generate a plurality of fatigue models depicting changes in │ ⌇
│  drivers' driving fatigue level over time at different vehicle │
│       speeds, based on a plurality of second samples      │
└─────────────────────────────────────────────────┘
                        │
                        ▼
┌─────────────────────────────────────────────────┐  S1134
│  Determine the driving distance when the driver experiences │ ⌇
│  fatigue in the middle portion of the tunnel using the fatigue │
│      model, based on the driving fatigue threshold and the │
│                    specified speed                │
└─────────────────────────────────────────────────┘
```

**FIG. 9**

Collect heart rate variability data of one of multiple experimental drivers in a calm state — S11311

Instruct the experimental driver to drive the vehicle into the tunnel at one of a plurality of experimental speeds, and collect the driver's heart rate variability data in real time — S11312

Record the moments when the experimental driver feels fatigued during the driving process, for determining the heart rate variability data of the driver at that moment — S11313

Repeat steps S11311 to S11313 for each experimental driver and each experimental speed — S11314

**FIG. 10**

**FIG. 11**

$L_{tr} = L_{th}(1.9+t)^{-1.4}$

$L_{th} = 100\%$

t= Passage Time

**FIG. 12**

3.4m

1.2m  10.95°

355m

60km/h

**FIG. 13A**

3.35m

1.2m  10.15°

377m

80km/h

**FIG. 13B**

4.4m

1.2m  8.95°

564m

100km/h

**FIG. 13C**

10.15°

60°  3.35m

**FIG. 13D**

**FIG. 14A**

**FIG. 14B**

FIG. 15

a）0.5cd/m$^2$

b）1cd/m$^2$

c）1.5cd/m$^2$

d）2cd/m$^2$

FIG. 16

a）Red

b）Orange

c）Yellow

d）Green

e）Cyan

f）Blue

g）Purple

FIG. 17

FIG. 18

FIG. 19

a）Blue　　　　　　　　b）Purple

c）Cyan

**FIG. 20**

**FIG. 21**

**FIG. 22**

<div style="text-align:center">**INTERNATIONAL SEARCH REPORT**</div>

| | International application No. |
|---|---|
| | **PCT/CN2023/089754** |

**A.    CLASSIFICATION OF SUBJECT MATTER**

A61B5/18(2006.01)i；  A61B5/024(2006.01)i；  A61M21/00(2006.01)i；  G06F17/10(2006.01)i；  E02D29/045(2006.01)i；
B60W40/08(2012.01)i

According to International Patent Classification (IPC) or to both national classification and IPC

**B.    FIELDS SEARCHED**

Minimum documentation searched (classification system followed by classification symbols)

IPC：  A61B A61M G06F E02D B60W

Documentation searched other than minimum documentation to the extent that such documents are included in the fields searched

Electronic data base consulted during the international search (name of data base and, where practicable, search terms used)

CNTXT; ENTXTC; WPABSC; VEN; ENTXT; CNKI; 百度学术, BAIDU SCHOLAR: 北京工业大学, 胡江碧, 高小娟, 郭云鹏, 隧道, 疲劳, 唤醒, 长度, 间距, 颜色, 亮度, 刺激量, 维持时间, tunnel, fatigue, awaken+, length, maintaining time, wake-up, colo?r?, brightness, stimulus amount, distance

**C.    DOCUMENTS CONSIDERED TO BE RELEVANT**

| Category* | Citation of document, with indication, where appropriate, of the relevant passages | Relevant to claim No. |
|---|---|---|
| PX | CN 114732410 A (BEIJING UNIVERSITY OF TECHNOLOGY) 12 July 2022 (2022-07-12) claims 1-18 | 1-20 |
| PX | 岳振 等 (YUE, Zhen et al.). "隧道驾驶疲劳唤醒段设置长度研究 (Setting Length of Driving Fatigue Arousal Section in a Tunnel)" 隧道建设(中英文) (Tunnel Construction (Chinese and English)), Vol. 42, No. 6, 20 June 2022 (2022-06-20), ISSN: 2096-4498, text, page 1015, right column, line 18 to page 1020, right column, line 29 | 1-11, 17-20 |
| A | CN 111028479 A (BEIJING UNIVERSITY OF TECHNOLOGY et al.) 17 April 2020 (2020-04-17) description, paragraphs [0004]-[0054], and figures 1-7 | 1-20 |
| A | CN 113795069 A (SHENZHEN AOXIN TECHNOLOGY CO., LTD.) 14 December 2021 (2021-12-14) entire document | 1-20 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **24 July 2023** | **07 August 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/089754** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2022097603 A1 (MITSUBISHI ELECTRIC CORP.) 31 March 2022 (2022-03-31)<br>       entire document | 1-20 |
| A | CN 105868570 A (BEIJING UNIVERSITY OF TECHNOLOGY) 17 August 2016<br>(2016-08-17)<br>       entire document | 1-20 |
| A | CN 107299602 A (WUHAN UNIVERSITY OF TECHNOLOGY) 27 October 2017<br>(2017-10-27)<br>       entire document | 1-20 |

Form PCT/ISA/210 (second sheet) (July 2022)

**INTERNATIONAL SEARCH REPORT**
Information on patent family members

International application No.

**PCT/CN2023/089754**

| Patent document cited in search report | | | Publication date (day/month/year) | Patent family member(s) | Publication date (day/month/year) |
|---|---|---|---|---|---|
| CN | 114732410 | A | 12 July 2022 | None | |
| CN | 111028479 | A | 17 April 2020 | None | |
| CN | 113795069 | A | 14 December 2021 | None | |
| US | 2022097603 | A1 | 31 March 2022 | None | |
| CN | 105868570 | A | 17 August 2016 | None | |
| CN | 107299602 | A | 27 October 2017 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210430058